# EUROPEAN PATENT APPLICATION

(11) **EP 2 732 753 A1**
(43) Date of publication of application: **21.05.2014**
(21) Application number: 13775071.7
(22) Date of filing: 09.04.2013
(51) Int. Cl.: A61B 1/00, G02B 23/24

(54) **SWITCH COVER, SWITCH DEVICE, AND ENDOSCOPE**

(30) Priority: 11.04.2012 JP 2012090227; 28.08.2012 JP 2012187849
(71) Applicant: Olympus Medical Systems Corp., Tokyo 151-0072 (JP)
(72) Inventor: HOSHINO, Yuki, Shibuya-ku Tokyo 151-0072 (JP)
(74) Representative: Schicker, Silvia
(86) International application number: PCT/JP2013/060727
(87) International publication number: WO 2013/154106

(57) **Abstract**

A switch cover 50 is configured by having a first deformation portion 53 that is connectively provided at a flat surface portion 52a of a cylindrical portion 52 placed to surround a periphery of a switch portion 41, and deforms by an external stress, a second deformation portion 54 that is connectively provided at a curved surface portion 52b of the cylindrical portion 52, and deformed by the external stress, and a stress transmitting portion 55 that is connectively provided at the first and the second deformation portions 53, 54, and transmits a stress having a component in a perpendicular direction with respect to an outer surface of an operation portion main body 23 to the switch portion 41 when the first deformation portion 53 is deformed inward of the cylindrical portion 52 by the external stress.

## Description

### Technical Field

The present invention relates to a switch cover which an operator presses and operates by a finger of a hand that grasps an operation portion, a switch apparatus, and an endoscope.

### Background Art

In general, an operation portion of a handheld device such as an endoscope is provided with a pressing type switch apparatus to cause the device itself or a peripheral device thereof to perform actuation or the like in a predetermined manner in response to operation input of an operator.

Here, for example, in the case of use of an endoscope that is a handheld device, an operator such as a surgeon generally grasps an insertion portion by a right hand, and grasps an operation portion with a left hand. Accordingly, when an operator presses and operates a switch apparatus that is provided at the operation portion, the operator needs to press and operate a desired switch apparatus by moving the thumb, the forefinger and the like of the left hand while keeping the state in which the operator grasps the operation portion with the left hand.

However, depending on the disposition of respective switch apparatuses on operation portions, and the sizes of the hands of operators, the fingers of the operators do not sufficiently reach all the switch apparatuses, and pressing and operating the switch apparatus in the vertical direction are difficult in some cases. In such cases, operability and operation efficiency are likely to be reduced in such a manner that the switch sections contained in the switch apparatuses are not actuated properly and the operators have to press and operate the switch apparatuses again after the operators grip the operation portions once again.

As the art for handling with the above case, and actuating the switch portion by not only the pressing operation from the vertical direction of the switch apparatus but also by the pressing operation from a horizontal direction and a diagonal direction, Japanese Patent Application Laid-Open Publication No. 5-211987, for example, discloses a push button apparatus (a switch apparatus) including a finger rest member (a switch cover) that protrudes sideward of an operation portion, a first stem to which a pressing force that works on the finger rest member is transmitted, a second stem provided connectively to the first stem, and a switch portion that is pressed by the second stem to perform switching action.

However, in the technique disclosed in Japanese Patent Application Laid-Open Publication No. 5-211987, in order to transmit the stress that is inputted to the finger rest member by the operator's pressing to the switch portion, a plurality of members such as first and second stems are required and therefore the structure is made complicated and the number of parts is increased. Thus, in the technique according to Japanese Patent Application Laid-Open Publication No. 5-211987, it takes time for assembly and manufacturing costs increase and also weight of the operation portion to be grasped by the operator increases to cause a case where the operator is burdened.

The present invention has been made in view of the above circumstances and an object of the present invention is to provide a switch cover, a switch apparatus, and an endoscope that are capable of actuating a switch portion with an extremely simple configuration by a pressing operation in the horizontal direction as well as a pressing operation in the vertical direction.

### Disclosure of Invention

### Means for Solving the Problem

A switch cover according to one aspect of the present invention is a switch cover of a pressing type switch apparatus having a switch portion that is contained in an operation portion of a handheld device to be operated by an operator and performs a switching action by a stress having a component in a perpendicular direction with respect to an outer surface of the operation portion, the switch cover including: a cylindrical portion that is placed to surround a periphery of the switch portion and extends in a direction substantially perpendicular to the outer surface of the operation portion over an inside and an outside of the operation portion; a flexible first deformation portion that is connectively provided at a partial region in a circumferential direction of the cylindrical portion on the outside of the operation portion, and deformed by an external stress that is given by pressing; a flexible second deformation portion that is connectively provided at a region other than the first deformation portion in the circumferential direction of the cylindrical portion on the outside of the operation portion, and deformed more easily than the first deformation portion by the external stress; and a stress transmitting portion that is connectively provided at the first deformation portion and the second deformation portion, and transmits the stress having the component in the perpendicular direction with respect to the outer surface of the operation portion to the switch portion when the first deformation portion is deformed inward of the cylindrical portion by the external stress.

Further, a switch apparatus according to one aspect of the present invention is a pressing type switch apparatus including a switch portion that is contained in an operation portion of a handheld device to be operated by an operator and performs a switching action by a stress having a component in a perpendicular direction with respect to an outer surface of the operation portion, and a switch cover that transmits the stress to the switch portion, wherein the switch cover includes a cylindrical portion that is placed to surround a periphery of the switch portion, and extends in a direction substantially perpendicular to the outer surface of the operation portion over an inside and an outside of the operation portion, a flexible first deformation portion that is connectively provided at a partial region in a circumferential direction of the cylindrical portion on the outside of the operation portion, and deformed by an external stress that is given by pressing, a flexible second deformation portion that is connectively provided at a region other than the first deformation portion in the circumferential direction of the cylindrical portion on the outside of the operation portion, and deformed more easily than the first deformation portion by the external stress, and a stress transmitting portion that is connectively provided at the first deformation portion and the second deformation portion, and transmits the stress having the component in the perpendicular direction with respect to the outer surface of the operation portion to the switch portion when the first deformation portion is deformed inward of the cylindrical portion by the external stress.

Further, an endoscope according to one aspect of the present invention is an endoscope provided with a pressing type switch apparatus including a switch portion that is contained in an operation portion to be operated by an operator and performs a switching action by a stress having a component in a perpendicular direction with respect to an outer surface of the operation portion, and a switch cover that transmits the stress to the switch portion, wherein the switch cover includes a cylindrical portion that is placed to surround a periphery of the switch portion, and extends in a direction substantially perpendicular to the outer surface of the operation portion over an inside and an outside of the operation portion, a flexible first deformation portion that is connectively provided at a partial region in a circumferential direction of the cylindrical portion on the outside of the operation portion, and deformed by an external stress that is given by pressing, a flexible second deformation portion that is connectively provided at a region other than the first deformation portion in the circumferential direction of the cylindrical portion on the outside of the operation portion, and deformed more easily than the first deformation portion by the external stress, and a stress transmitting portion that is connectively provided at the first deformation portion and the second deformation portion, and transmits the stress having the component in the perpendicular direction with respect to the outer surface of the operation portion to the switch portion when the first deformation portion is deformed inward of the cylindrical portion by the external stress.

### Brief Description of the Drawings

Fig. 1 is a view showing an endoscope system according to an embodiment of the present invention;
Fig. 2 is an explanatory view of an occasion in which an operation portion of an endoscope is grasped with a left hand and a switch operation is performed according to the embodiment of the present invention;
Fig. 3 is a perspective view showing an operation portion main body according to the embodiment of the present invention;
Fig. 4 is a side view showing the operation portion main body according to the embodiment of the present invention;
Fig. 5 is a sectional view of an essential part taken along line V-V of Fig. 4 according to the embodiment of the present invention;
Fig. 6 is a sectional view of an essential part taken along line VI-VI of Fig. 4 according to the embodiment of the present invention;
Fig. 7 is an enlarged sectional view of the essential part of Fig. 5 according to the embodiment of the present invention;
Fig. 8 is a perspective view showing a switch cover unit from a front surface side according to the embodiment of the present invention;
Fig. 9 is a perspective view showing the switch cover unit from a back surface side according to the embodiment of the present invention;
Fig. 10 is a sectional view of an essential part showing a deformed state of the switch cover with respect to a pressing operation from a substantially horizontal direction according to the embodiment of the present invention;
Fig. 11 is a sectional view of the essential part showing a deformed state of the switch cover with respect to a pressing operation from a substantially vertical direction according to the embodiment of the present invention;
Fig. 12 is a sectional view of an essential part showing a modification of the switch cover according to the embodiment of the present invention;
Fig. 13 is a sectional view of an essential part showing a modification of the switch cover according to the embodiment of the present invention;
Fig. 14 is a plan view showing a modification of the switch cover according to the embodiment of the present invention;
Fig. 15 is a schematic view showing an endoscope system according to a second embodiment of the present invention;
Fig. 16 is a schematic view showing an operation portion of an endoscope of the endoscope system according to the second embodiment of the present invention;
Fig. 17A is a schematic sectional view showing a switch portion of the operation portion of the endoscope of the endoscope system according to the second embodiment of the present invention;
Fig. 17B is an enlarged view of a position indicated by 17B in Fig. 17A according to the second embodiment of the present invention;
Fig. 18A is a front view of an outer sheath case of the switch portion of the operation portion of the endoscope of the endoscope system according to the second embodiment of the present invention;
Fig. 18B is a left side view taken along the arrow 18B line in Fig. 18A according to the second embodiment of the present invention;
Fig. 18C is a right side view taken along the arrow 18C line in Fig. 18A according to the second embodiment of the present invention;
Fig. 18D is a schematic perspective view of the outer sheath case in Fig. 18A seen from a diagonally lower left of a front side according to the second embodiment of the present invention;
Fig. 18E is a schematic perspective view of the outer sheath case of Fig. 18A seen from a diagonally lower right of the front side according to the second embodiment of the present invention;
Fig. 19A is a front view of a flexible plate of the switch portion of the operation portion of the endoscope of the endoscope system according to the second embodiment of the present invention;
Fig. 19B is a left side view taken along the arrow 19B line in Fig. 19A according to the second embodiment of the present invention;
Fig. 19C is a right side view taken along the arrow 19C line in Fig. 19A according to the second embodiment of the present invention;
Fig. 19D is a schematic perspective view of a flexible plate in Fig. 19A seen from a diagonally lower left of a front side according to the second embodiment of the present invention;
Fig. 19E is a schematic perspective view of the flexible plate in Fig. 19A seen from a diagonally lower right of the front side according to the second embodiment of the present invention;
Fig. 19F is a schematic view taken along the arrow 19F line in Fig. 19A according to the second embodiment of the present invention;
Fig. 19G is a schematic view taken along the arrow 19G line in Fig. 19A according to the second embodiment of the present invention;
Fig. 20A is a front view of an intermediate member of the switch portion of the operation portion of the endoscope of the endoscope system according to the second embodiment of the present invention;
Fig. 20B is a left side view taken along the arrow 20B line in Fig. 20A according to the second embodiment of the present invention;
Fig. 20C is a right side view taken along the arrow 20C line in Fig. 20A according to the second embodiment of the present invention;
Fig. 20D is a schematic perspective view of an intermediate member in Fig. 20A seen from a diagonally lower left of a front side according to the second embodiment of the present invention;
Fig. 20E is a schematic perspective view of the intermediate member in Fig. 20A seen from a diagonally lower right of the front side according to the second embodiment of the present invention;
Fig. 20F is a schematic view taken along the arrow 20F line in Fig. 20A according to the second embodiment of the present invention;
Fig. 21A is a front view of a holder of the switch portion of the operation portion of the endoscope of the endoscope system according to the second embodiment of the present invention;
Fig. 21B is a left side view taken along the arrow 2 1 B line in Fig. 21 A according to the second embodiment of the present invention;
Fig. 21C is a right side view taken along the arrow 21C line in Fig. 21 A according to the second embodiment of the present invention;
Fig. 21D is a schematic perspective view of a holder in Fig. 21A seen from a diagonally lower left of a front side according to the second embodiment of the present invention;
Fig. 21E is a schematic perspective view of the holder in Fig. 21A seen from a diagonally lower right of the front side according to the second embodiment of the present invention;
Fig. 22A is a front view of a board of the switch portion of the operation portion of the endoscope of the endoscope system according to the second embodiment of the present invention;
Fig. 22B is a left side view taken along the arrow 22B line in Fig. 22A according to the second embodiment of the present invention;
Fig. 22C is a right side view taken along the arrow 22C line in Fig. 22A according to the second embodiment of the present invention;
Fig. 22D is a schematic perspective view of the board in Fig. 22A seen from a diagonally lower left of a front side according to the second embodiment of the present invention;
Fig. 22E is a schematic perspective view of the board in Fig. 22A seen from a diagonally lower right of the front side according to the second embodiment of the present invention;
Fig. 23A is an enlarged view of a position indicated by 17B in Fig. 17A of an endoscope of an endoscope system according to a modification of the second embodiment of the present invention;
Fig. 23B is an enlarged view of the position indicated by 17B in Fig. 17A of the endoscope of the endoscope system according to the modification of the second embodiment of the present invention;
Fig. 23C is an enlarged view of the position indicated by 17B in Fig. 17A of the endoscope of the endoscope system according to the modification of the second embodiment of the present invention;
Fig. 24A is a front view of a flexible plate of a switch portion of an operation portion of the endoscope of the endoscope system according to a modification of the second embodiment of the present invention;
Fig. 24B is a left side view taken along the arrow 24B line in Fig. 24A according to the modification of the second embodiment of the present invention;
Fig. 24C is a right side view taken along the arrow 24C line in Fig. 24A according to the modification of the second embodiment of the present invention;
Fig. 24D is a schematic perspective view of the flexible plate in Fig. 24A seen from a diagonally lower left of a front side according to the modification of the second embodiment of the present invention;
Fig. 24E is a schematic perspective view of the flexible plate in Fig. 24A seen from a diagonally lower right of the front side according to the modification of the second embodiment of the present invention;
Fig. 25 is a schematic sectional view showing a state in which the flexible plate of the operation portion of the endoscope of the endoscope system and the intermediate member are integrally molded according to a modification of the second embodiment of the present invention;
Fig. 26A is a schematic sectional view of a switch portion of an operation portion of an endoscope of an endoscope system according to a modification of the second embodiment of the present invention; and
Fig. 26B is an enlarged view of a position indicated by 26B in Fig. 26A according to the modification of the second embodiment of the present invention.

### Best Mode for Carrying Out the Invention

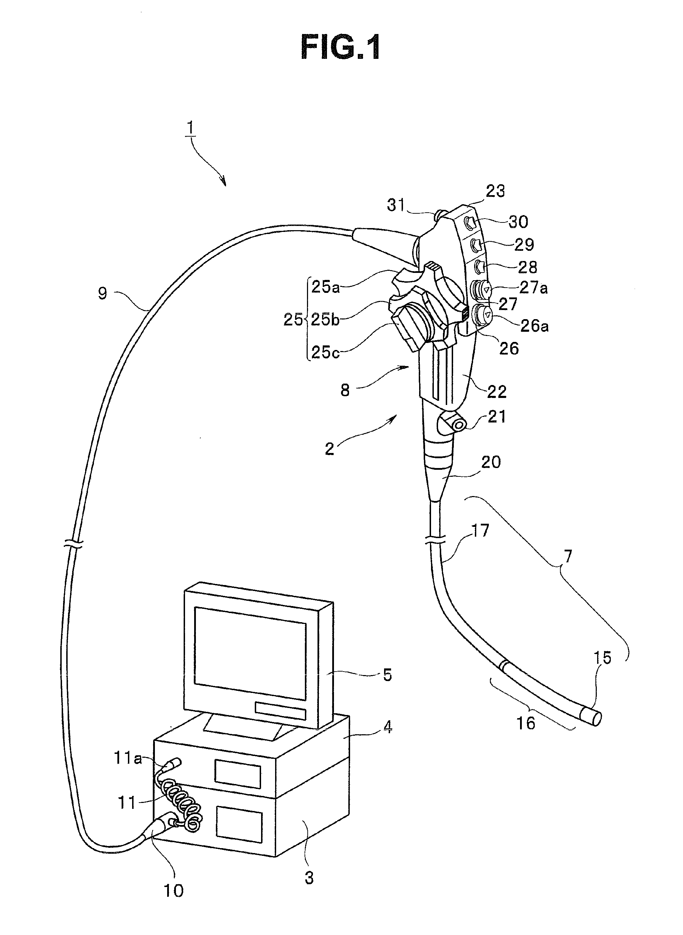

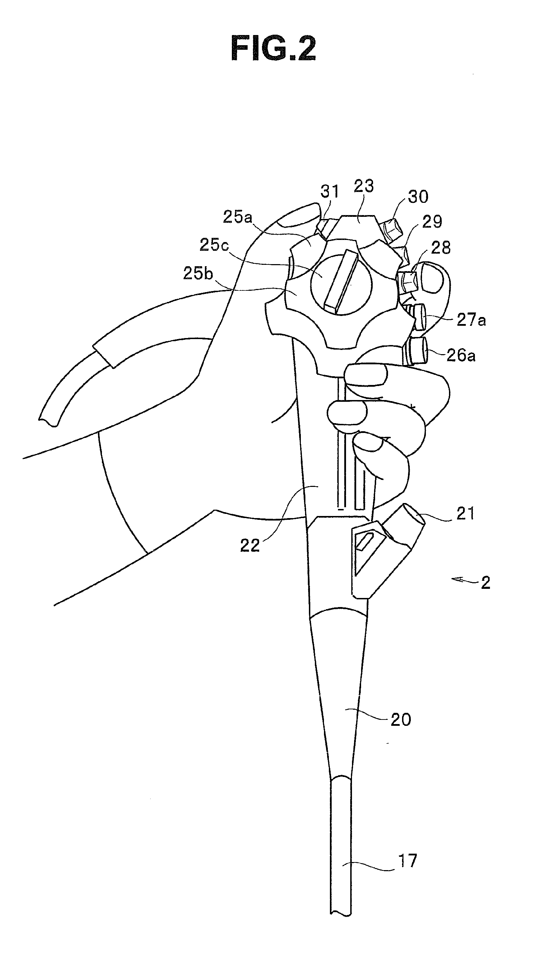

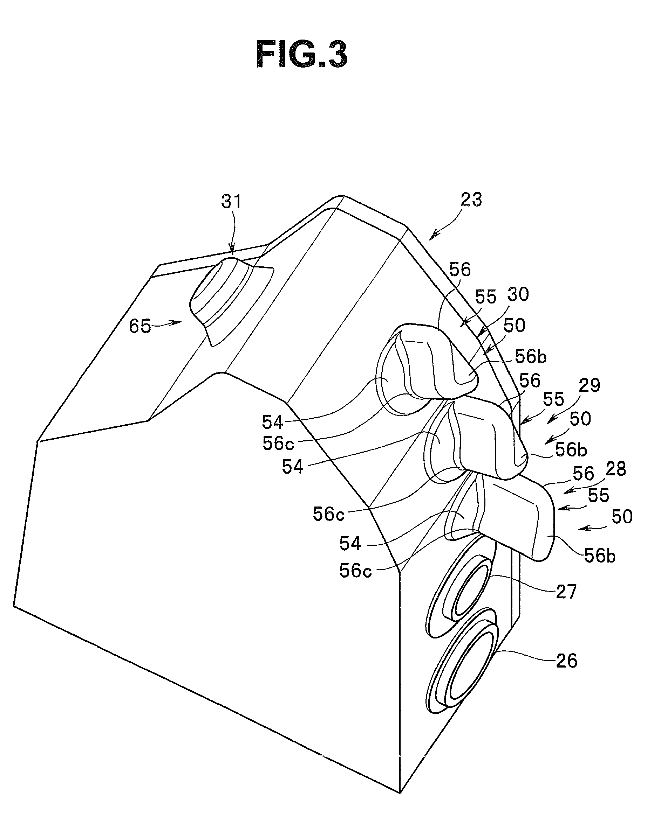

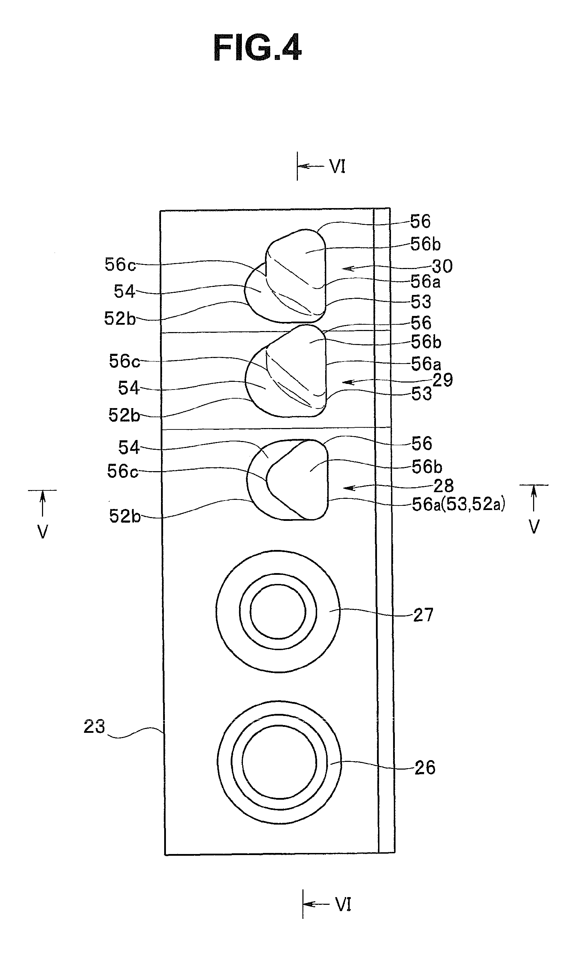

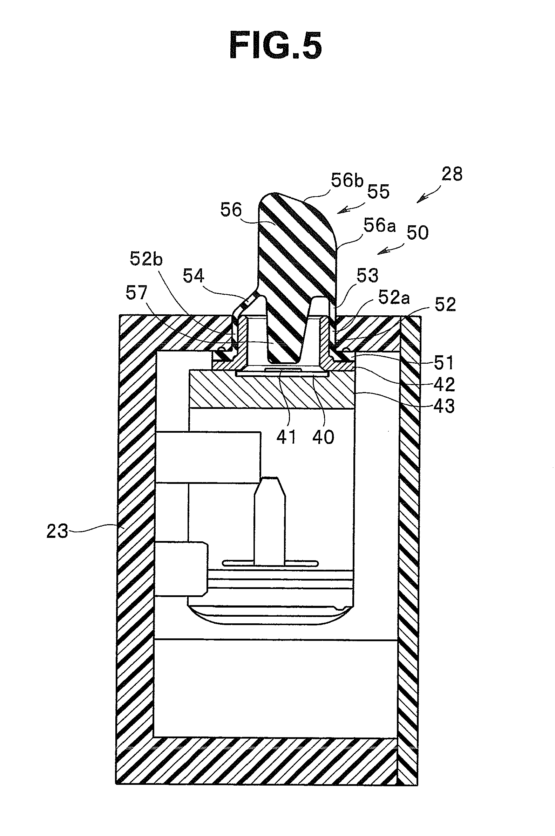

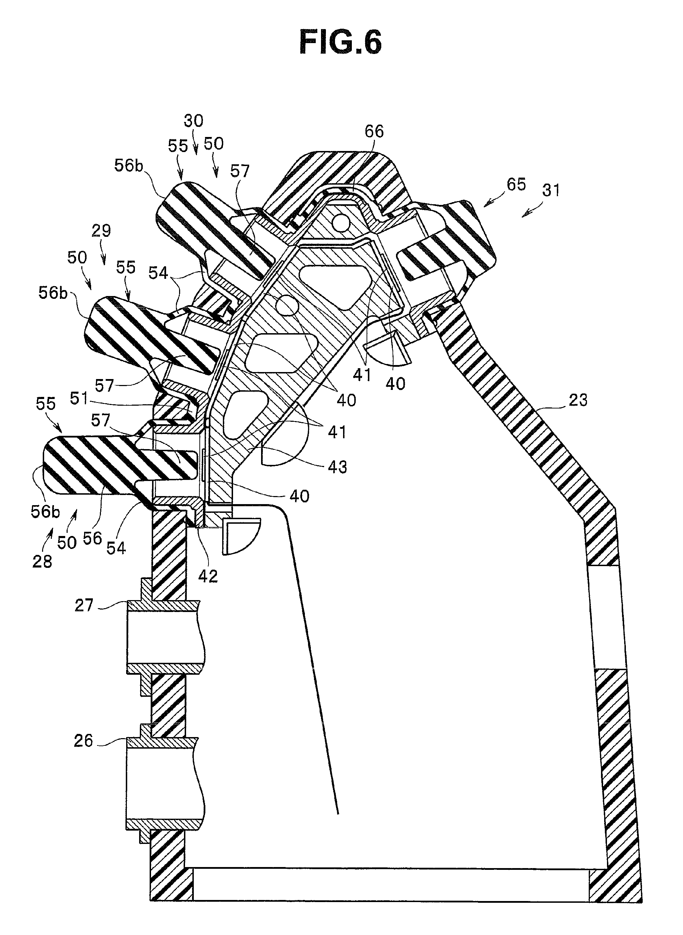

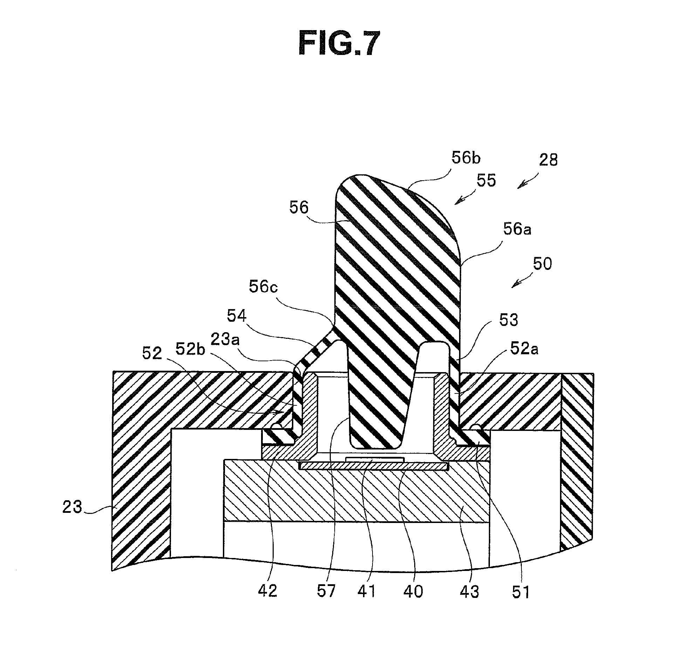

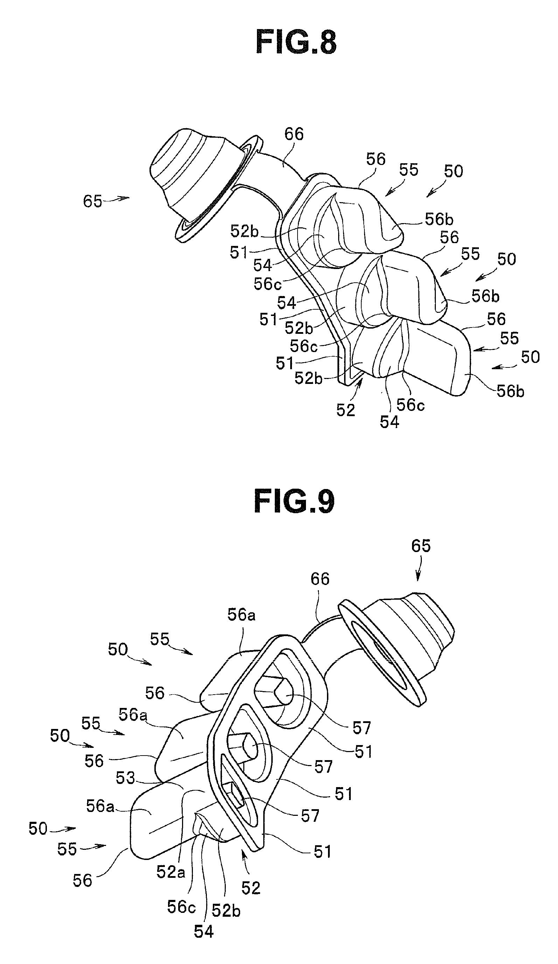

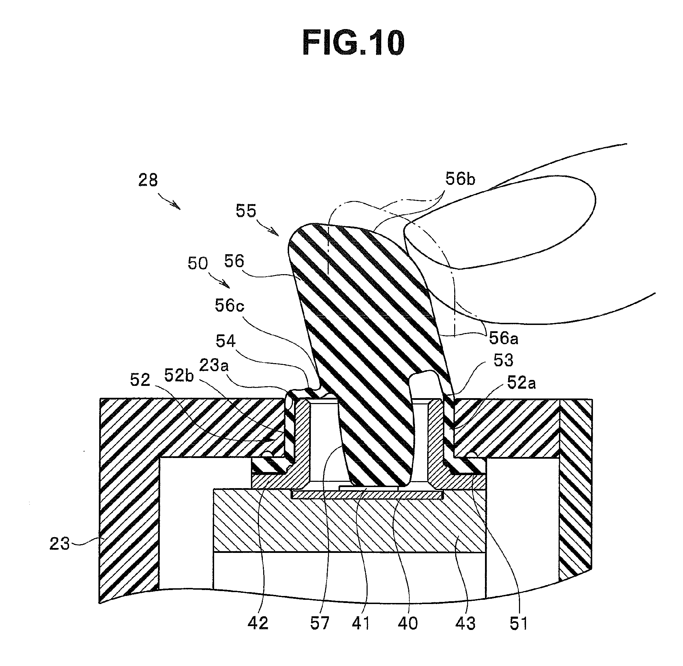

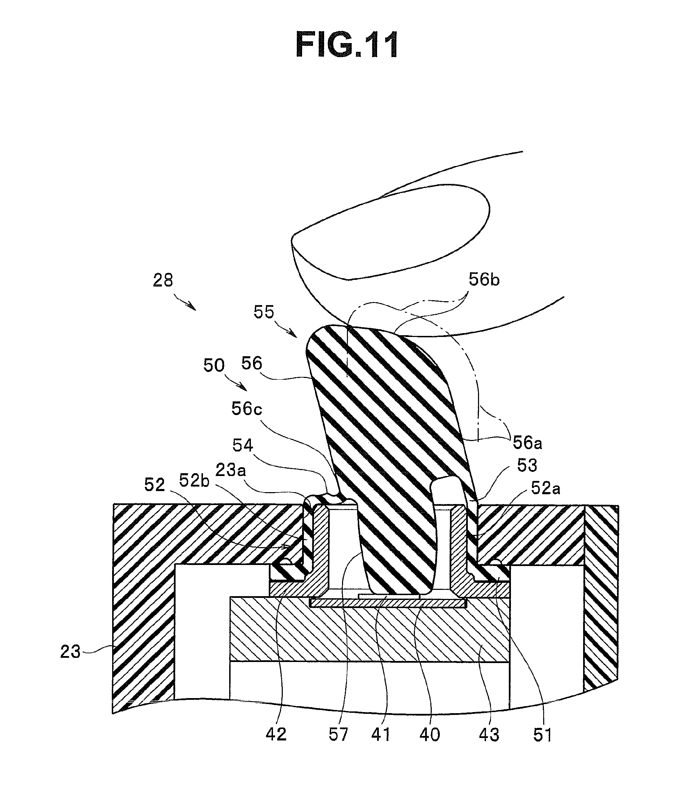

Hereinafter, modes of the present invention will be described with reference to the drawings. The drawings relate to one embodiment of the present invention. Fig. 1 is a view showing an endoscope system. Fig. 2 is an explanatory view of an occasion when an operation portion of an endoscope is grasped with a left hand and switch operation is performed. Fig. 3 is a perspective view showing an operation portion main body. Fig. 4 is a side view showing the operation portion main body. Fig. 5 is a sectional view of an essential part taken along line V-V of Fig. 4. Fig. 6 is a sectional view of an essential part taken along line VI-VI of Fig. 4. Fig. 7 is an enlarged sectional view of the essential part of Fig. 5. Fig. 8 is a perspective view showing a switch cover unit from a front surface side. Fig. 9 is a perspective view showing the switch cover unit from a back surface side. Fig. 10 is a sectional view of an essential part showing a deformed state of the switch cover with respect to a pressing operation from a substantially horizontal direction. Fig. 11 is a sectional view of the essential part showing a deformed state of the switch cover with respect to a pressing operation from a substantially vertical direction.

As shown in Fig. 1, an endoscope system 1 of the present embodiment has a main part configured by having an endoscope 2 that is one example of a handheld device, a light source apparatus 3, a video processor 4 that is a CCU (a camera control unit), and a monitor 5.

As shown in the drawing, the endoscope 2 is configured by having an insertion portion 7 as an elongated hollow long member that is inserted into a site to be observed, an operation portion 8 that is provided connectively at a proximal end portion of the insertion portion 7, a universal cable 9 that is provided extensively from a side surface portion of the operation portion 8, a light source connector 10 that is provided at an extension end portion of the universal cable 9, an electric cable 11 that is extended from a side portion of the light source connector 10, and an electric connector 11a that is placed at an extension end of the electric cable 11. Note that the light source connector 10 is detachably connected to the light source apparatus 3, and the electric connector 11a is detachably connected to the video processor 4.

The insertion portion 7 has a distal end portion 15 at a distal end side, and a bending portion 16 as a bendable movable portion is connectively provided at a proximal end portion of the distal end portion 15. Further, a flexible tube portion 17 that is formed by a tubular member having flexibility, and has a long length and flexibility is connectively provided at a proximal end portion of the bending portion 16.

The operation portion 8 is configured by having a bend preventing portion 20 that is connected to a proximal end side of the insertion portion 7, a treatment instrument insertion port 21 that is placed in a vicinity of the bend preventing portion 20 and communicates with a treatment instrument insertion channel in the insertion portion 7, a grasping portion 22 that is placed at a proximal portion side from the treatment instrument insertion port 21, and an operation portion main body 23 that is connectively provided at a proximal end side of the grasping portion 22.

The operation portion main body 23 is provided with a bending operation knob 25 for operating the bending portion 16 of the insertion portion 7. The bending operation knob 25 has a UD bending operation knob 25a for performing a bending operation of the bending portion 16 in up and down directions, and an RL bending operation knob 25b for performing a bending operation of the bending portion 16 in left and right directions, and the UD bending operation knob 25a and the RL bending operation knob 25b are disposed by being superimposed on each other to be rotatable on the same axis. Further, a release knob 25c that is operated when rotation of the respective bending operation knobs 25a and 25b are stopped/released is provided in a center portion of the RL bending operation knob 25b.

Furthermore, the operation portion main body 23 is provided with, for example, an air/water feeding cylinder 26 that communicates with a water feeding tank (not illustrated), a suction cylinder 27 that communicates with a suction tank (not illustrated), and four pressing type N switch apparatuses (remote switches) 28 to 31. A detachable air/water feeding button 26a is mounted to the air/water feeding cylinder 26. A detachable suction button 27a is mounted to the suction cylinder 27. Further, the respective switch apparatuses 28 to 31 are properly assigned with an image freezing function of freezing an image of the endoscope, a function of transmitting image data to a printer, various functions for adjusting an endoscope image and the like.

Here, as shown in, for example, Fig. 1 to Fig. 4, the air/water feeding cylinder 26, the suction cylinder 27 and the three switch apparatuses 28 to 30 are disposed in a single line along a longitudinal axis direction of the operation portion 8, in one side surface portion at a side where the treatment instrument insertion port 21 is opened. Further, the remaining one switch apparatus 31 is disposed on the other side surface portion (the side surface portion at an opposite side from the aforementioned one side surface portion) at a side where the universal cable 9 extends.

The light source apparatus 3 supplies an illuminating light to a light guide not illustrated that is provided in the endoscope 2. Namely, in the universal cable 9, the operation portion 8 and the insertion portion 7 of the endoscope 2 of the present embodiment, a light guide not illustrated is placed, and via the light guide, the light source apparatus 3 supplies the illuminating light to an illumination optical system (not illustrated) that configures an illuminating window of a distal end portion 6. A subject site is irradiated with the illuminating light by the illumination optical system. Note that the aforementioned water feeding tank is attached to the light source apparatus 3, and a water feeding pump (not illustrated) that feeds sterilized water to the endoscope 2 from the water feeding tank is further contained.

The video processor 4 properly converts the image data that is picked up by the endoscope 2 into a video signal based on the operation signals from the switch apparatuses 28 to 31, for example, and causes the monitor 5 to display the image data.

Incidentally, at the time of use of the endoscope system 1 configured as above, the operator such as a surgeon usually grasps the insertion portion 7 with a right hand, and grasps the operation portion 8 with a left hand. In this case, grasping to the operation portion 8 is basically realized by gripping the grasping portion 22 with use of a middle finger, a ring finger and a little finger of a left hand from the side where the universal cable 9 is extended, as shown in Fig. 2, for example. When the operation portion 8 is grasped in this manner, the operator can operate the air/water feeding button 26a, the suction button 27a and the switch apparatuses 28 to 30 mainly by the forefinger of the left hand, and can operate the switch apparatus 31 by a thumb. At this time, in particular, switching action of the switch apparatuses 28 to 30 that are located far from the grasping portion 22 can be performed by not only a pressing operation from the vertical direction, but also by a pressing operation from a horizontal direction.

Hereinafter, a specific configuration of the switch apparatuses 28 to 30 will be described. Note that in the following explanation, with respect to the components common to the respective switch apparatuses 28 to 30, only the components of the switch apparatus 28 will be described by representing them, the components of the switch apparatuses 29 and 30 will be assigned with the same reference signs, and explanation thereof will be properly omitted.

As shown in Figs. 5 to 7, the switch apparatus 28 is configured by having a switch board 40 that is placed in the operation portion main body 23, and a switch cover 50 that transmits an external stress by a switch operation of an operator from an outside of the operation portion main body 23 to the switch board 40.

Note that though detailed explanation will be omitted, the switch apparatus 31 also has the switch board 40 similar to those of the switch apparatuses 28 to 30, and further has a switch cover 65 that transmits an external stress by a switch operation of the operator from an outside of the operation portion main body 23 to the switch board 40.

The switch board 40 has a pressing type normally open switch portion 41 in which two contact points (not illustrated) are disposed by being superimposed, on the board, in a state in which the two contact points (not illustrated) are separated from each other, for example.

Here, a switch opening portion 23a corresponding to the switch apparatus 28 is provided in a side surface portion of the operation portion main body 23, and the switch board 40 is placed substantially parallel along an inner wall surface of the operation portion main body 23, in a state in which the switch portion 41 is caused to face an inside of the switch opening portion 23a. Further, between the switch board 40 and the operation portion main body 23, a washer 42 that is fitted in the switch opening portion 23a via the switch cover 50 is provided, and the switch board 40 is sandwiched between the washer 42, and a support member 43 that is fixedly provided in the operation portion main body 23. Thereby, the switch board 40 is held in the operation portion main body 23, and the switch portion 41 can perform switching action by an external stress having a component in a perpendicular direction to an outer surface of the operation portion main body 23 (the operation portion 8).

The switch cover 50 has, for example, a proximal portion 51 in a flat shape, a cylindrical portion 52 perpendicularly provided from the proximal portion 51, a first deformation portion 53 and a second deformation portion 54 that are connectively provided at an end portion of the cylindrical portion 52, and a stress transmission portion 55 that are connectively provided at end portions of the first and the second deformation portions 53 and 54, and has a main part configured by these portions being integrally molded from a soft resin or the like.

Note that, for example, as shown in Figs. 8 and 9, in the present embodiment, the switch covers 50 of the respective switch apparatuses 28 to 30 are connected to one another via the proximal portions 51, and further are also connected to a switch cover 65 of the switch apparatus 31 via a connection portion 66. Namely, in the present embodiment, the respective switch covers 50 of the switch apparatuses 28 to 30, and the switch cover 65 of the switch apparatus 31 are configured by an integral resin molded product or the like.

As shown in Figs. 8 and 9, for example, the cylindrical portion 52 is configured by a cylindrical body with a flat surface portion 52a and a curved surface portion 52b mainly combined. The cylindrical portion 52 is interposed between the switch opening portion 23a of the operation portion main body 23 and the washer 42 in a state in which the cylindrical portion 52 surrounds a periphery of the switch portion 41. Thereby, the cylindrical portion 52 is extended in a direction substantially perpendicular to the outer surface of the operation portion main body 23 throughout an inside and an outside of the operation portion main body 23.

The first deformation portion 53 is connectively provided in a part of a region in a circumferential direction of the cylindrical portion 52, in an exterior of the operation portion main body 23. More specifically, in the present embodiment, the first deformation portion 53 is formed by a flat surface portion continuing to the flat surface portion 52a of the cylindrical portion 52.

The second deformation portion 54 is connectively provided in a region except for the first deformation portion 53 in the circumferential direction of the cylindrical portion 52, in an exterior of the operation portion main body 23. More specifically, in the present embodiment, the second deformation portion 54 is formed by a tapered surface portion that continues to the curved surface portion 52b of the cylindrical portion 52. Namely, in contrast with the first deformation portion 53 that is configured by the surface portion that extends without substantially inclining to the perpendicular direction to the outer surface of the operation portion main body 23, the second deformation portion 54 is configured by a surface portion that inclines at a larger angle than the first deformation portion 53 to the perpendicular direction to the outer surface of the operation portion main body 23. In other words, the second deformation portion 54 is configured by the surface portion that inclines at a predetermined elevation angle to a first deformation portion 53 side. By the second deformation portion 54 inclining like this, a stepped shape is formed between the second deformation portion 54 and the cylindrical portion 52 (the curved surface portion 52b), and thereby, the second deformation portion 54 can be more easily deformed than the first deformation portion 53.

Here, as shown in, for example, Figs. 4 and 8, in a circumferential direction of the cylindrical portion 52, a region where the second deformation portion 54 is formed is set to be a region longer than a region where the first deformation portion 53 is formed.

As shown in Figs. 5 to 7, for example, the stress transmission portion 55 is configured by having a protrusive operation input portion 56 that continues to end portions of the first and the second deformation portions 53 and 54, and a pressing portion 57 that transmits the external stress that is inputted into the operation input portion 56 to the switch portion 41.

The operation input portion 56 is configured by, for example, a solid member that forms a substantially triangular pole shape with a sectional area in an inner circumferential direction thereof relatively smaller than a sectional area in an inner circumferential direction of the cylindrical portion 52.

Among three flat surfaces that configure a circumferential face of the operation input portion 56, one flat surface is set as a first pressed surface 56a capable of inputting the external stress by pressing of the operator. The first pressed surface 56a configures the same flat surface as the flat surface portion 52a of the cylindrical portion 52 and the first deformation portion 53. Namely, in the present embodiment, the operation input portion 56 is disposed to be offset to the first deformation portion 53 side with respect to a center of the cylindrical portion 52, whereby the first pressed surface 56a is disposed to be flush with the flat surface portion 52a of the cylindrical portion 52, and the first deformation portion 53.

Further, a top surface of the operation input portion 56 is set as a second pressed surface 56b capable of inputting the external stress by pressing of the operator. The second pressed surface 56b is formed by an inclined surface having a predetermined elevation angle to the second deformation portion 54 side from the first deformation portion 53 side. The second pressed surface 56b is provided with the inclination like this, whereby even when the external stress from a longitudinal axis direction of the operation input portion 56 is inputted through the second pressed surface 56b, the stress component in the direction perpendicular to the first pressed surface 56a can be generated.

Among the three flat surfaces that configure the circumferential face of the operation input portion 56, the two remaining flat surfaces are connectively provided at the end portion of the second deformation portion 54 by having a predetermined angle. Thereby, a comer-shaped portion 56c that is formed by the remaining two flat surfaces of the operation input portion 56 is caused to face a substantially center of an end portion of the second deformation portion 54.

The pressing portion 57 is configured by a rod-shaped member integrally provided at a proximal end portion of the operation input portion 56. The pressing portion 57 is disposed in a space that is formed by the cylindrical portion 52, and the first and the second deformation portions 53 and 54, and a distal end portion thereof is disposed in a position where the distal end portion is contactable to and separable from the switch portion 41 to face the switch portion 41. Note that the pressing portion 57 of the present embodiment is formed integrally with the operation input portion 56, but may be configured such that the pressing portion 57 formed by a separate member is integrally provided fixedly at the proximal end portion of the operation input portion 56, for example.

Next, an operation of the switch apparatus 28 including the switch cover 50 configured as above will be described.

When the first pressed surface 56a of the switch apparatus 28 is pressed by a forefinger of the operator who grasps the grasping portion 22 with a left hand, as shown in Figs. 2 and 10, for example, the external stress in the horizontal direction to the outer surface of the operation portion main body 23 is mainly inputted to the operation input portion 56. Thereby, first, the second deformation portion 54 that is more easily deformed than the first deformation portion 53 starts buckling deformation with the stepped shape or the like with the cylindrical portion 52 (the curved surface portion 52b) as a starting point. Thereupon, with the buckling deformation of the second deformation portion 54 as an impetus, the first deformation portion 53 starts flexing deformation to the second deformation portion 54 side (an inside of the cylindrical portion 52).

Subsequently, by deformation of the first and the second deformation portions 53 and 54, the operation input portion 56 tilts in the direction of the second deformation portion 54. Namely, deformation of the second deformation portion 54 is buckling deformation, whereas deformation of the first deformation portion 53 is flexing deformation, and therefore, the operation input portion 56 tilts in the direction of the second deformation portion 54 with the first deformation portion 53 as a supporting point.

With the tilt of the operation input portion 56, an end portion of the pressing portion 57 is caused to abut on the switch portion 41, and transmits a stress having a component in the perpendicular direction to the outer surface of the operation portion main body 23 to the switch portion 41. Thereby, the two contact points not illustrated of the switch portion 41 are electrically connected, and the switch apparatus 28 is turned on.

Note that when the first pressed surface 56a is released from pressing, the operation input portion 56 is raised by restoring forces of the first and the second deformation portions 53 and 54, and with this, the switch apparatus 28 is turned off.

Further, when the second pressed surface 56b of the switch apparatus 28 is pressed, as shown in Fig. 11, for example, the external stress in the perpendicular direction to the outer surface of the operation portion main body 23 is mainly inputted into the operation input portion 56, and a part thereof is converted into a stress component in the horizontal direction to the outer surface of the operation portion main body 23 by the inclination of the second pressed surface 56b. Thereby, the second deformation portion 54 that is more easily deformed than the first deformation portion 53 firstly starts buckling deformation with the stepped shape or the like with the cylindrical portion 52 (the curved surface portion 52b) as the starting point, similarly to the time when the first pressed surface 56a is pressed. Thereupon, the first deformation portion 53 starts flexing deformation with the buckling deformation of the second deformation portion 54 as an impetus.

Subsequently, by deformation of the fist and the second deformation portions 53 and 54, the operation input portion 56 tilts in the direction of the second deformation portion 54. Namely, deformation of the second deformation portion 54 is buckling deformation, whereas deformation of the first deformation portion 53 is flexing deformation, and therefore, the operation input portion 56 tilts in the direction of the second deformation portion 54 with the first deformation portion 53 as the supporting point.

With the tilt of the operation input portion 56, the end portion of the pressing portion 57 is caused to abut on the switch portion 41, and transmits the stress having the component in the perpendicular direction to the outer surface of the operation portion main body 23 to the switch portion 41. Thereby, the two contact points not illustrated of the switch portion 41 are electrically connected, and the switch apparatus 28 is turned on.

Note that when the second pressed surface 56b is released from pressing, the operation input portion 56 is raised by the restoring forces of the first and the second deformation portions 53 and 54, and with this, the switch apparatus 28 is turned off.

According to the embodiment as above, the switch cover 50 is configured by having the first deformation portion 53 that is connectively provided at the flat surface portion 52a of the cylindrical portion 52 that is placed to surround the periphery of the switch portion 41, and is deformed by an external stress, the second deformation portion 54 that is connectively provided at the curved surface portion 52b of the cylindrical portion 52, and is deformed by the external stress, and the stress transmission portion 55 that is connectively provided at the first and the second deformation portions 53 and 54, and transmits a stress having a component in the perpendicular direction to the outer surface of the operation portion main body 23 to the switch portion 41 when the first deformation portion 53 is deformed inward of the cylindrical portion 52 by the external stress, whereby the switch portion 41 can be actuated not only by the pressing operation from the perpendicular direction to the operation input portion 56, but also by the pressing operation from the horizontal direction, by the extremely simple configuration.

In the above case, the second deformation portion 54 is formed by the inclined portion (the tapered surface portion) that inclines at a larger angle than the first deformation portion 53 with respect to the perpendicular direction to the outer surface of the operation portion main body 23, whereby the second deformation portion 54 can be buckled and deformed with respect to an external stress, and even when the first and the second deformation portions 53 and 54 are formed with the same wall thickness, for example, the second deformation portion 54 can be deformed more easily than the first deformation portion 53. Furthermore, if the stepped shape is formed between the second deformation portion 54 and the cylindrical portion 52 (the curved surface portion 52b) by inclination of the second deformation portion 54, the second deformation portion 54 can be more effectively buckled and deformed.

Further, for example, the operation input portion 56 is formed into a substantially triangular pole shape, and the comer-shaped portion 56c of the two flat surfaces configuring the circumferential face of the operation input portion 56 is caused to face the end portion of the second deformation portion 54, whereby the comer-shaped portion 56c can be caused to function as a stress concentration portion that causes an external stress to concentrate on a part of the second deformation portion 54, and at the time of input of the external stress, the second deformation portion 54 can be buckled and deformed more properly.

Further, the sectional area of the operation input portion 56 is set to be the sectional area smaller than the sectional area of the cylindrical portion 52, and the operation input portion 56 is disposed by being offset to the first deformation portion 53 side with respect to the center of the cylindrical portion 52, whereby the release region at the time of buckling deformation of the second deformation portion 54 can be set, and the second deformation portion 54 can be buckled and deformed more properly.

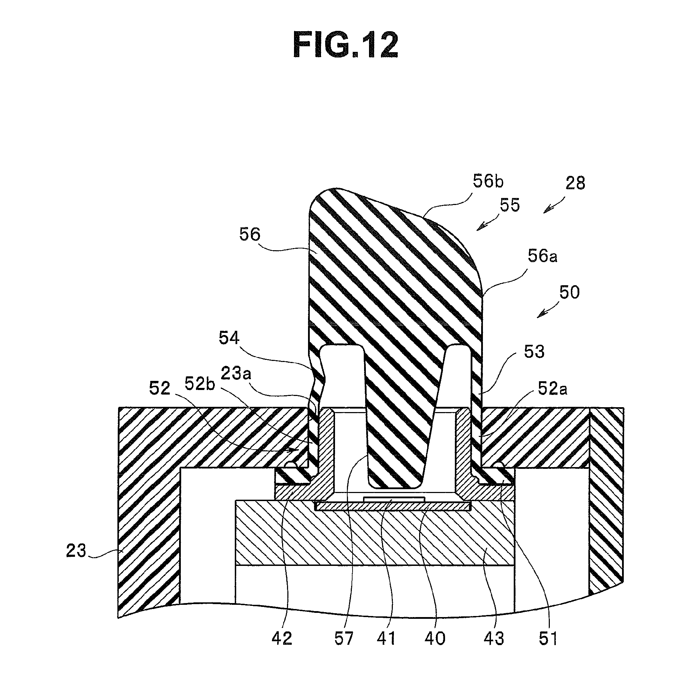

Here, in the aforementioned embodiment, one example in which the entire region of the second deformation portion 54 is configured by the inclined surface portion is described, but the present invention is not limited to the example, and as shown in Fig. 12, for example, only a part of the second deformation portion 54 can be also configured by an inclined surface portion.

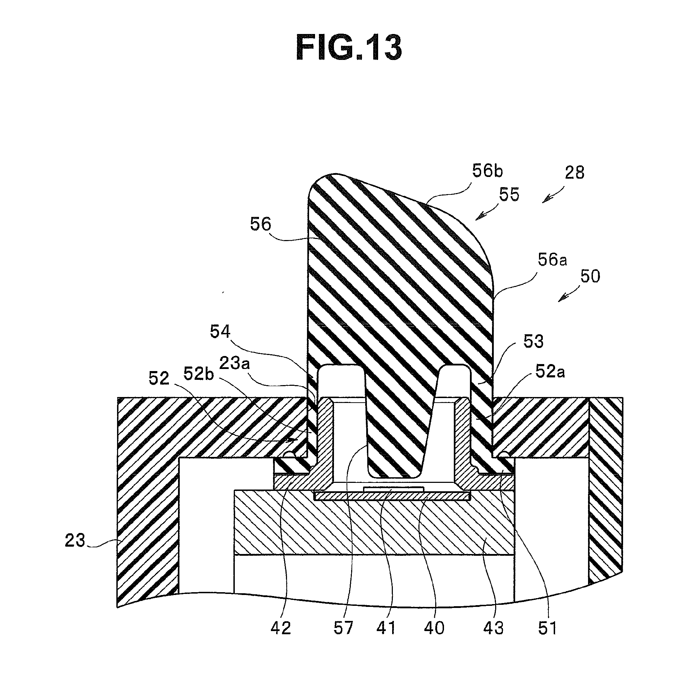

Further, in the aforementioned embodiment, one example of the configuration that deforms the second deformation portion 54 more easily than the first deformation portion 53 by configuring the second deformation portion 54 by the inclined surface portion is described, but the present invention is not limited to the example, and as shown in Fig. 13, for example, a configuration can be adopted, which deforms the second deformation portion 54 more easily than the first deformation portion 53 by forming a wall thickness of the second deformation portion 54 relatively thinner than a wall thickness of the first deformation portion 53.

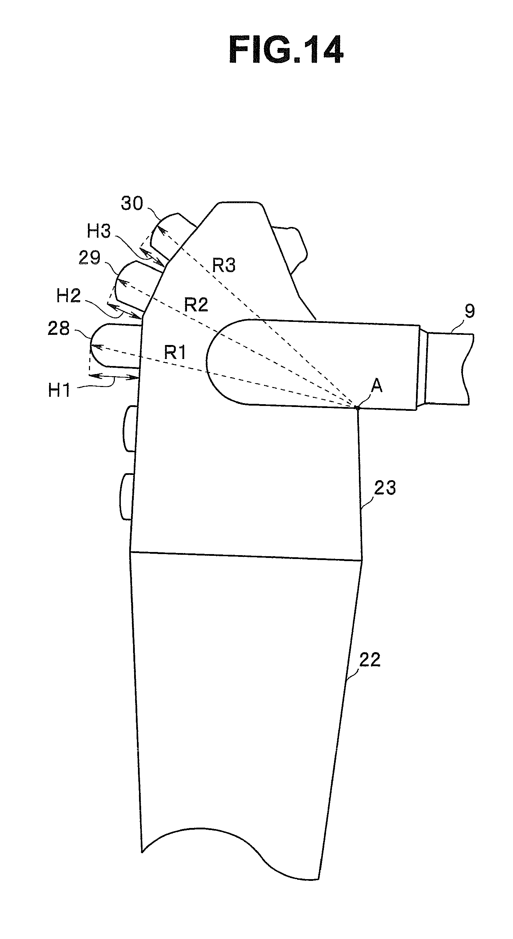

Further, in order to enhance operability to the respective switch apparatuses 28 to 30, for example, when the operation portion 8 of the endoscope 2 is grasped with a left hand, and an operation is performed with a root portion A of a portion where the universal cable 9 is attached to the operation portion main body 23, placed on a portion of a fork between a thumb and a forefinger, distances R1 to R3 from the root portion A to peaks of the respective switch apparatuses 28 to 30 can be configured to be equal to one another, as shown in Fig. 14.

With the configuration as above, the plurality of switch apparatuses 28 to 30 can be properly pressed without laboring by only a forefinger without the grasping hand being changed (without redoing grip).

Further, at this time, for the purpose of reducing the size of the operation portion main body 23, and for the purpose of causing the other switch apparatuses 28 to 30 to be easily pressed with a middle finger, the respective switch apparatuses 28 to 30 may be disposed in such a manner that distances H1 to H3 from the operation portion main body 23 to the respective peaks of the plurality of switch apparatuses 28 to 30 are not constant (for example, H1 > H2 > H3).

Note that the present invention is not limited to the respective embodiments described above, but various modifications and changes can be made, and the various modifications and the changes are also within the technical range of the present invention. For example, the handheld device to which the switch cover and the switch apparatus of the present invention is applied is not limited to an endoscope, but application to various handheld devices in which switch operations are performed with hands that keep grasping the operation portions as a matter of course.

Incidentally, concerning the endoscope having an operation portion operated by being grasped with a hand, Japanese Patent Application Laid-Open Publication No. 8-191789, for example, discloses that a plurality of electronic button switches (pressing portions) such as a release button are disposed at the operation portion of an endoscope. These button switches respectively have switch members containing switch main bodies, and operation members which the finger of an operator touches. In the operation member, a seat is integrally formed at a lower portion of a rubber cover in a substantially circular column shape. At a lower portion of the seat, two prismatic engaging portions are formed toward an inside of the operation portion. The engaging portions are engaged with locking holes of a circumferential edge portion of the switch attaching hole that is formed in the outer sheath case of the operation portion. A switch base and a switch receiver of the switch member, and the engaging portions are fastened by being screwed by a fixing screw.

In the structure of Japanese Patent Application Laid-Open Publication No. 8-191789, the button switches (the pressing portions) are separately attached to the outer sheath case respectively. Further, in the structure of Japanese Patent Application Laid-Open Publication No. 8-191798, each of the individual electronic button switches needs to be fastened by being screwed by a fixing screw. Accordingly, in the structure of Japanese Patent Application Laid-Open Publication No. 8-191798, assembly of the electronic button switches to the operation portion is complicated.

A second embodiment of the present invention is made to solve the problem as above, and has an object to provide an endoscope in which assemblability of a pressing portion to an operation portion is enhanced.

The endoscope according to the second embodiment includes an operation portion that is operated in a state grasped by a hand of a user, and an insertion portion that is extended from the operation portion, and is inserted into a pore, wherein the operation portion has an outer sheath case that has a first and a second inner circumferential faces that face each other, and a plurality of openings that are placed in at least one of the first and the second inner circumferential faces, and is grasped with the hand of the user, a flexible plate that has a plurality of pressing portions that are protruded through the openings to an outside from an inside of the outer sheath case and are elastically deformable, and is disposed along the first and the second inner circumferential faces of the outer sheath case, a plate-shaped intermediate member that is disposed in the inside of the flexible plate along the first and the second inner circumferential faces of the outer sheath case, and supports the pressing portions of the flexible plate respectively to prevent a liquid from penetrating into between the outer sheath case and the flexible plate, and a holder that holds a board having a switch that is switchable to a position where the switch is pressed by the pressing portion and a position where the switch is released with respect to the pressing portion, in a position facing the pressing portion, presses the intermediate member and the flexible plate by the intermediate member toward the first and the second inner circumferential faces of the outer sheath case to bring the intermediate member into close contact with the flexible plate and bring the flexible plate into close contact with the first and the second inner circumferential faces, and is supported in the inside of the outer sheath case.

The three members that are the flexible plate having the plurality of pressing portions, the intermediate member, and the holder that holds the board are attached to the outer sheath case, whereby the endoscope having the operation portion capable of using the plurality of pressing portions can be formed. Therefore, assemblability of the switch portion can be significantly enhanced. Further, the intermediate member that supports the pressing portions and prevents a liquid from penetrating into between the outer sheath case and the flexible plate is disposed between the flexible plate and the holder. Consequently, the function of preventing a liquid from penetrating into between the outer sheath case and the flexible plate while preventing excessive deformation of the pressing portions can be realized by the two members that are the flexible plate and the intermediate member being pressed to the outer sheath case with the holder.

Further, the intermediate member preferably has a ring-shaped edge portion that holds a state in which the pressing portion of the flexible plate is caused to face the board that is held by the holder, and preferably has ring-shaped engaging portions that are provided at the ring-shaped edge portion, and a proximal portion of the pressing portion of the flexible plate that faces the ring-shaped edge portion to be engaged with each other to restrict deformation of the proximal portion of the pressing portion.

The ring-shaped engaging portions that restrict deformation of the proximal portion of the pressing portion are formed between the intermediate member and the flexible plate, whereby when a large force is applied to the pressing portion, the position of the pressing portion can be kept.

Further, at least one of the inner circumferential face of the outer sheath case, a front surface or a back surface of the flexible plate, and a front surface of the intermediate member preferably has a ring-shaped convex portion that brings the front surface of the flexible plate and the inner circumferential face of the outer sheath case into close contact with each other to achieve water tightness between the front surface of the flexible plate and the inner circumferential face of the outer sheath case.

The ring-shaped convex portion is formed on at least one of the inner circumferential face of the outer sheath case, the front surface or the back surface of the flexible plate and the front surface of the intermediate member, whereby water tightness between the outer sheath case and the flexible plate is enhanced, and a liquid can be prevented from penetrating in therebetween.

Further, the intermediate member is formed from a raw material more rigid than the flexible plate, and is formed from the raw material more flexible than the outer sheath case and the holder.

When the flexible plate, the intermediate member and the holder are disposed with respect to the outer sheath case, the intermediate member that is more flexible than the holder, and the flexible plate that is more flexible than the intermediate member are pressed to the outer sheath case that is more rigid as compared with the flexible plate and the intermediate member, with the holder. Therefore, when the holder is disposed with respect to the outer sheath case, the flexible plate and the intermediate member can be easily deformed, and therefore, special work is not required at the time of assembly.

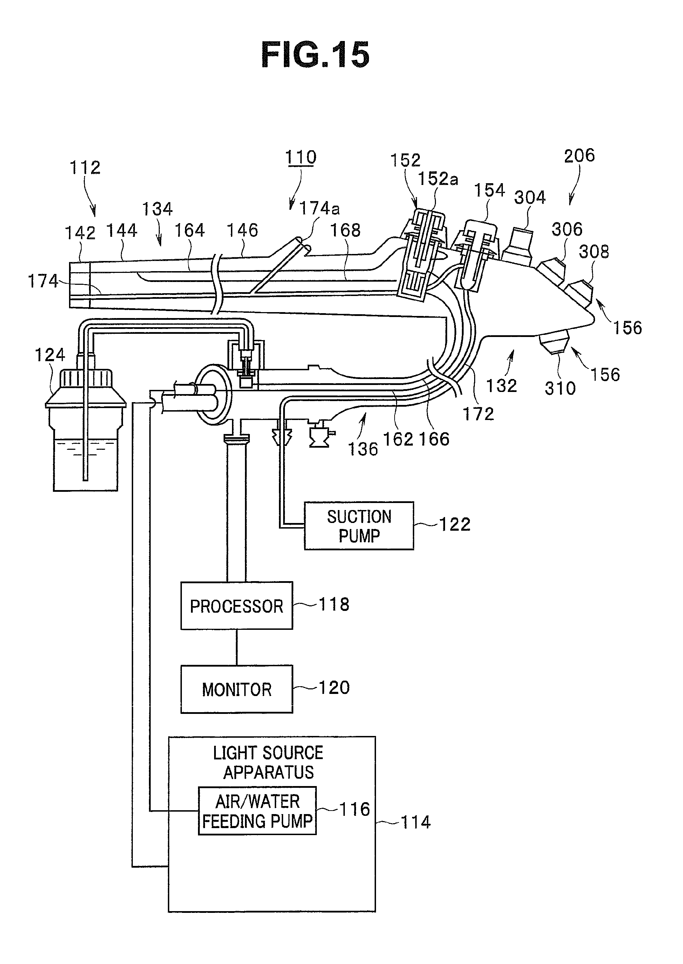

Hereinafter, with reference to Fig. 15 to Fig. 22, the second embodiment of the present invention will be described.

An endoscope system 110 according to the embodiment has an endoscope 112, a light source apparatus 114 having an air/water feeding pump 116, a processor 118, a monitor 120, a suction pump 122 and a water feeding tank 124.

The endoscope 112 according to the embodiment has an operation portion 132 that is operated in a state in which the operation portion 132 is grasped by a hand of a user, and an insertion portion 134 that is extended from the operation portion 132 and is inserted into a pore in a body cavity or the like. From the operation portion 132, a universal cable 136 is extended. To an extension end portion of the universal cable 136, the light source apparatus 114, the processor 118, the suction pump 122 and the water feeding tank 124 are connected.

The insertion portion 134 has a distal end rigid portion 142, a bending portion 144, and a flexible or rigid tube-shaped portion 146. At the distal end rigid portion 142, respective distal ends of a known illumination optical system and a known observation optical system are disposed, so that an object to be observed of a living body or the like, for example, can be illuminated and observed. In the bending portion 144, a bending operation knob not illustrated that is disposed at the operation portion 132 is rotated, whereby a distal end of the insertion portion 134 can be caused to bend in a desired direction with use of a known bending mechanism.

In the operation portion 132, an air/water feeding button 152, a suction button 154 and a plurality of electric switches 156 are provided side by side.

The air/water feeding button 152 is a part of a known air/water feeding mechanism. When the air/water feeding button 152 is not pressed down (namely, in the case of a non-operational state), an air supply tube 162 of the air/water feeding pump 116 shown in Fig. 15 is inserted through external air via a leak hole 152a of the air/water feeding button 152. Therefore, air is not fed to the air feeding tube 164. Further, in the state, the air supply tube 162 and the water feeding tank 124 are shut off. Therefore, in the non-operational state, both air feeding and water feeding are not performed from a distal end of the insertion portion 134.

If the air/water feeding button 152 is not pressed down and the leak hole 152a is closed with a finger, the leak hole 152a is closed, and therefore, the air supply tube 162 is caused to communicate with the air feeding tube 164. Thereby, air that is supplied to the air supply tube 162 flows into the air feeding tube 164, and air is fed (air is discharged) from the distal end of the insertion portion 134.

In an operational state in which the air/water feeding button 152 is pressed down, a water supply tube 166 and a water feeding tube 168 are caused to communicate with each other. Further, in the state, the air supply tube 162 and the air feeding tube 164 are shut off. Accordingly, the air supply tube 162 is shut off from other conduits, and therefore, the air supplied from the air/water feeding pump 116 is fed onto a liquid surface of the water feeding tank 124. Thereby, an internal pressure of the water feeding tank 124 rises, and a physiological saline solution of the water feeding tank 124 is fed to the water supply tube 166. Thereby, the physiological saline solution flows to the water feeding tube 168 from the water supply tube 166, and a liquid is fed from the distal end of the insertion portion 134 (the physiological saline solution is discharged).

In a non-operational state in which the suction button 154 is not pressed down, a suction tube 172 and a treatment instrument insertion channel 174 are shut off from each other. Accordingly, a suction operation is not performed in the distal end of the insertion portion 134.

In an operation state in which the suction button 154 is pressed down, the suction tube 172 and the treatment instrument insertion channel 174 are caused to communicate with each other. Accordingly, a negative pressure suction force of the suction tube 172 is transmitted to the treatment instrument insertion channel 174, and therefore suction can be performed from the distal end of the insertion portion 134.

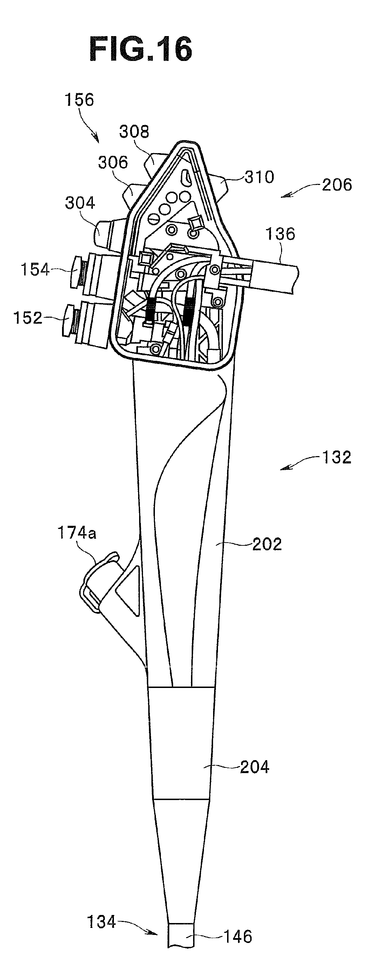

As shown in Fig. 16, the operation portion 132 has a grasping portion (grip) 202 that is grasped by a user, a bend preventer 204 that fixes a proximal end of the insertion portion 134 and prevents the proximal end of the insertion portion 134 from being buckled, and a switch portion 206 in which the air/water feeding button 152, the suction button 154 and a plurality of electric switches 156 are provided side by side. The switch portion 206 is disposed at a distant end opposite to the distal end of the insertion portion 134. A forceps plug 174a that is disposed at the grasping portion 202 is placed at a proximal end of the treatment instrument insertion channel 174 (see Fig. 15) which causes various treatment instruments not illustrated to protrude from the distal end of the distal end rigid portion 142 of the insertion portion 134. Note that in an inside of the insertion portion 134 and insides of the bend preventer 204 and the grasping portion 202 of the operation portion 132, parts of a known illumination optical system, a known observation optical system, a known bending mechanism, a known air/water feeding mechanism, and a known suction mechanism, for example, are placed.

Note that the aforementioned air/water feeding button 152 that performs switching of air feeding/water feeding of the air/water feeding mechanism, the aforementioned suction button 154 that performs switching of suction of the suction mechanism, and the plurality of electric switches 156 are respectively placed at an outer sheath case 212 that will be described later of the switch portion 206 of the operation portion 132.

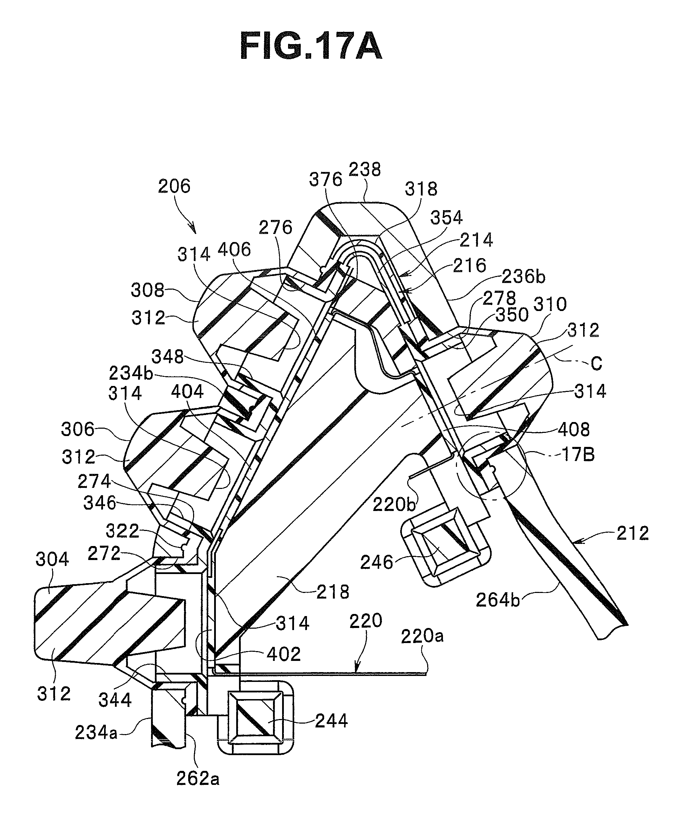

As shown in Fig. 17A, the switch portion 206 has the outer sheath case 212, a flexible plate 214, a plate-shaped intermediate member 216, and a holder 218 that holds a board 220 of the electric switch 156. In the embodiment, the outer sheath case 212 is assumed to be fixed to the grasping portion 202 shown in Fig. 16 detachably and water-tightly. Note that it is also preferable that the outer sheath case 212 is integrally formed at the grasping portion 202.

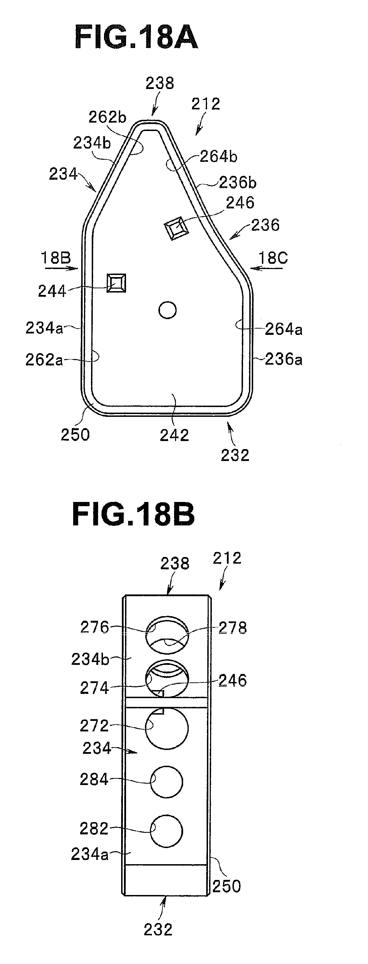

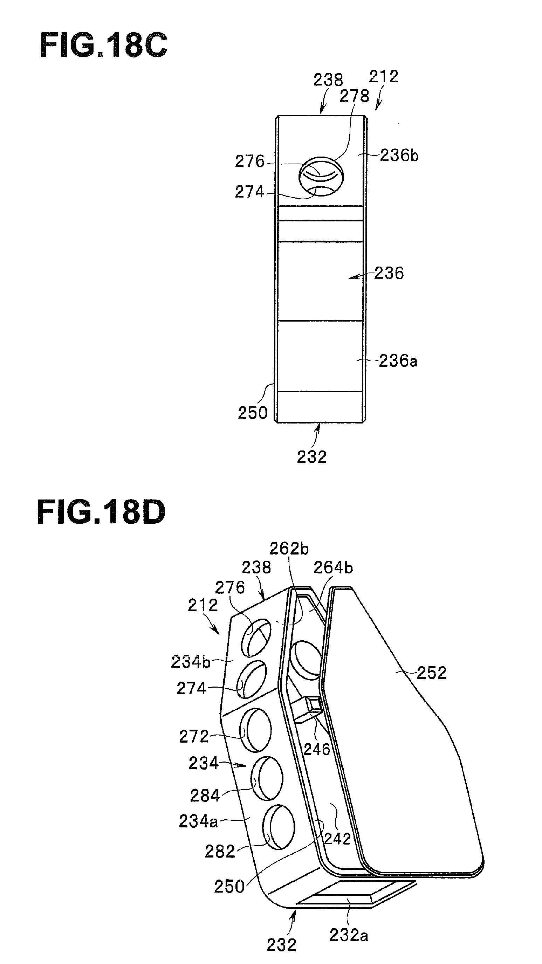

The outer sheath case 212 shown in Fig. 18A to Fig. 18E is formed from a rigid material having heat resistance, chemical resistance and electrical insulating properties, such as a denatured PPE (polyphenylene ether), for example.

The outer sheath case 212 has a proximal portion 232 that is attachable to and detachable from the grasping portion 202, and a first and a second case side portions 234 and 236 that are integrally extended from the proximal portion 232. The first and the second case side portions 234 and 236 are integrally connected at a top portion 238 that faces the proximal portion 232. Namely, the proximal portion 232, the first and the second case side portions 234 and 236, and the top portion 238 are cooperatively formed into a substantially cylindrical shape.

Note that in the proximal portion 232, an opening 232a through which the aforementioned tubes 164, 168 and 174 (see Fig. 15), electric cables not illustrated of the electric switch portions 156 and the like are passed is formed. Note that an image pickup device of an observation optical system is electrically connected to an insertion portion side electric cable not illustrated of the electric switch portion 156. Further, for example, in the second case side portion 236, an opening not illustrated which allows the tubes 162, 166 and 172 (see Fig. 15), the electric cables not illustrated of the electric switch portions 156 and the like to pass through and to which the proximal end of the universal cable 136 is connected is formed. Electric cables for peripheral apparatuses not illustrated of the electric switch portions 156 are electrically connected to the peripheral apparatuses such as the light source apparatus 114 and the processor 118. Note that an opening to which the universal cable 136 is fixed is preferably formed at a second case raised portion 23 6a that will be described later of the second case side portion 236, for example.

The first case side portion 234 has a first case raised portion 234a that is raised substantially perpendicularly to the proximal portion 232, and a first case slant surface portion 234b that is inclined toward the top portion 238 from the first case raised portion 234a. The second case side portion 236 has the second case raised portion 236a that is raised substantially perpendicularly to the proximal portion 232, and a second case slant surface portion 236b that is inclined toward the top portion 238 from the second case raised portion 236a. In the embodiment, a width of the outer sheath case 212 is preferably formed to be gradually smaller toward the top portion 238 along the first and the second case side portions 234 and 236 from the proximal portion 232 by the first and the second case slant surface portions 234b and 236b.

A bottom portion 242 as a side wall of the outer sheath case 212 is integrally formed in a substantially cylindrical portion that is formed by the proximal portion 232, the first and the second case side portions 234 and 236 and the top portion 238. On the bottom portion 242, two columns (a first and a second columns) 244 and 246 that hold the holder 218 at a predetermined position are formed in a state extended toward an opening 250 of the aforementioned substantially cylindrical portion. Sections of the columns 244 and 246 are formed to be rectangular in the embodiment, but may be in various shapes such as circles. A lid member 252 shown in Fig. 18D is fixed to the opening 250 of the substantially cylindrical portion in a watertight state.

As shown in Fig. 18A, an inner circumferential face 262a of the first case raised portion 234a, and an inner circumferential face 264a of the second case raised portion 236a face each other. Here, the inner circumferential faces 262a and 264a are preferably parallel with each other, for example. An inner circumferential face (a first inner circumferential face) 262b of the first case slant surface portion 234b, and an inner circumferential face (a second inner circumferential face) 264b of the second case slant surface portion 236b are inclined at appropriate angles to face each other in a substantially V shape or the like, for example. Note that the first case slant surface portion 234b and the second case slant surface portion 236b may form an acute angle, a right angle, and an obtuse angle. In the embodiment, explanation is made on the precondition that an acute angle is formed.

The first case raised portion 234a has a first electric switch opening 272 in which one pressing portion 304 that is integrally formed at the flexible plate 214 is placed. The first case slant surface portion 234b has a second and a third electric switch openings 274 and 276 in which two pressing portions 306 and 308 that are integrally formed at the flexible plate 214 are placed. The second case slant surface portion 236b has a fourth electric switch opening 278 in which one pressing portion 310 that is integrally formed at the flexible plate 214 is placed. The first electric switch opening 272 is adjacent to the second electric switch opening 274.

Note that the first case raised portion 234a has an air/water feeding button opening 282 in which the air/water feeding button 152 is placed, and a suction button opening 284 in which the suction button 154 is placed.

Namely, in the first case side portion 234, the five openings 272, 274, 276, 282 and 284 are formed, and among the openings, the three openings that are provided side by side at a side in close vicinity to the top portion 238 with respect to the proximal portion 232 are the electric switch openings 272, 274 and 276.

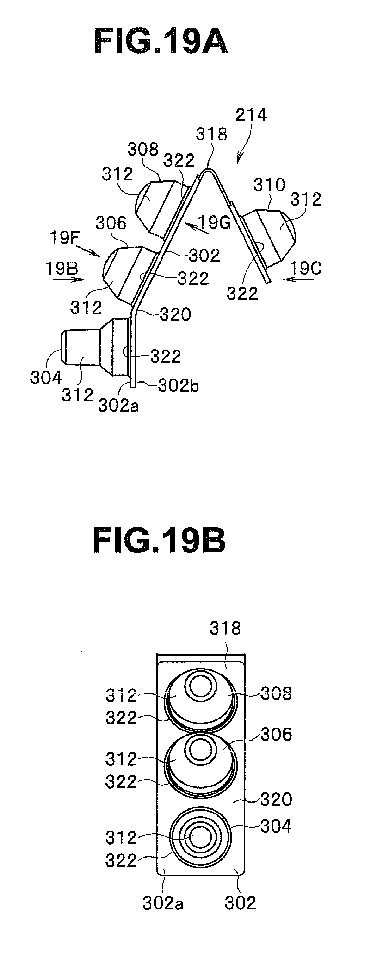

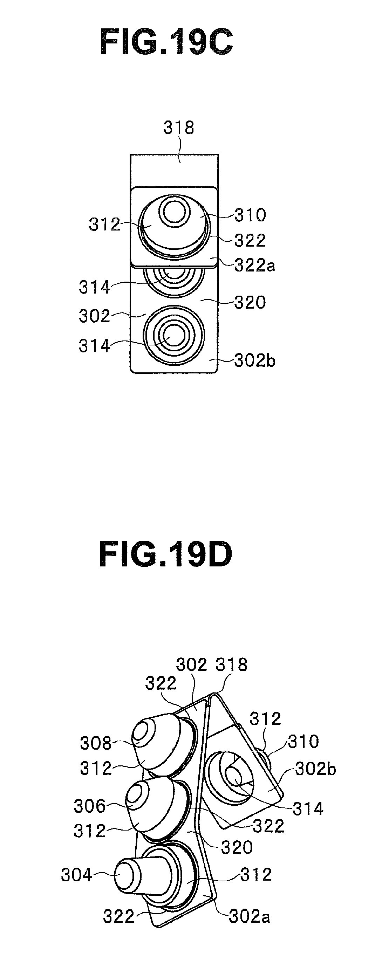

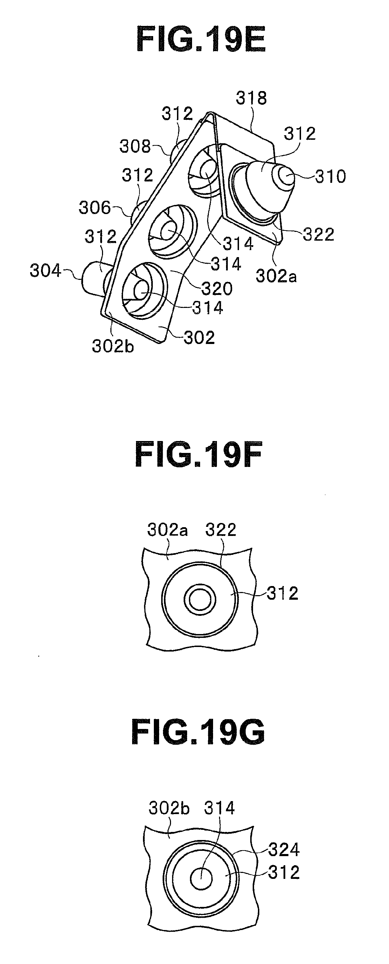

The flexible plate 214 shown in Fig. 19A to Fig. 19E are formed from, for example, a flexible resin material having heat resistance and electrical insulating properties such as a silicon resin material. Namely, the flexible plate 214 is a sufficiently flexible material with respect to the outer sheath case 212.

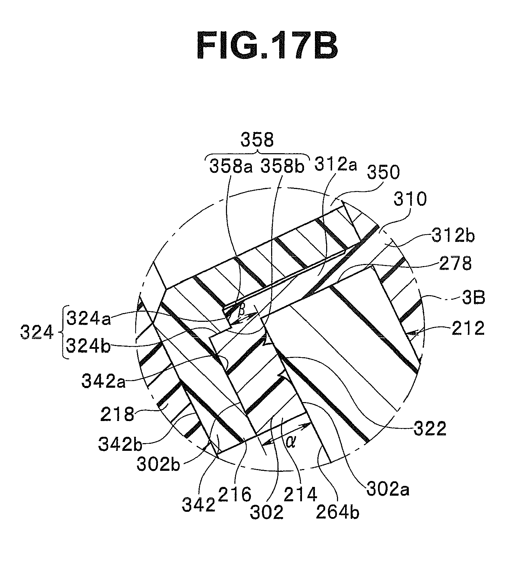

The flexible plate 214 has a flexible plate portion 302, and the four pressing portions (the first to the fourth pressing portions) 304, 306, 308 and 310 in the embodiment. The pressing portions 304, 306, 308 and 310 protrude to a front surface 302a side of the flexible plate portion 302. The respective pressing portions 304, 306, 308 and 310 each have a button portion 312 that is integrally formed at the flexible plate portion 302 and has a proximal portion 312a, and a convex portion 314 that is integrally formed at a deformation portion 312b of the button portion 312 to face the board 220. Here, as shown in Fig. 17B, the button portions 312 are formed by the proximal portions 312a that are sandwiched respectively between a first to a fourth collars 344, 346, 348 and 350 that will be described later and the first to the fourth electric switch openings 272, 274, 276 and 278, and the deformation portions 312b at sides protruded to the outer surface of the outer sheath case 212 along a center axis C with respect to the first to the fourth collars 344, 346, 348 and 350.

The respective pressing portions 304, 306, 308 and 310 are preferably formed to give click sensing to the operator when the operator presses the respective pressing portions. Note that all the respective pressing portions 304, 306, 308 and 310 may be in the same shapes, or may be in different shapes. In the embodiment, the first pressing portion 304 in close vicinity to the suction button 154 shown in Fig. 15 is formed into a different shape from the other pressing portions (the second to the fourth pressing portions) 306, 308 and 310 in the same shapes as one another.

The front surface 302a of the flexible plate portion 302 can be in close contact with the inner circumferential face 262a of the first case raised portion 234a, the inner circumferential face 262b of the first case slant surface portion 234b, and the inner circumferential face 264b of the second case slant surface portion 236b, of the outer sheath case 212. In particular, the inner circumferential faces 262b and 264b of the first and the second case slant surface portions 234b and 236b are formed to have acute angles to each other, and therefore, a folded portion 318 for allowing the flexible plate portion 302 to be easily folded is preferably formed at the flexible plate portion 302. Note that the folded portion 318 is preferably formed by being thinned, but a raw material with higher flexibility than other sites of the flexible plate portion 302 may be used.

Further, of the flexible plate portion 302, a site (a boundary) corresponding to the first case raised portion 234a and the first case slant surface portion 234b has a folded portion 320 that is foldable. Since with respect to an inclination angle between the inner circumferential face 262b of the first slant surface portion 234b and the inner circumferential face 264b of the second slant surface portion 236b, an obtuse angle is formed, the folded portion 320 can be easily curved without processing such as thin wall processing being applied, due to flexibility of the flexible plate portion 302.

Of the flexible plate portion 302 around the respective button portions 312, at positions (the front surface 302a side) at protruding direction sides of the respective pressing portions 304, 306, 308 and 310, ring-shaped protrusions 322 shown in Fig. 19A to Fig. 19F that are caused to abut on the flat inner circumferential faces 262a, 262b and 264b of the outer sheath case 212 are formed. The flexible plate portion 302 of the flexible plate 214 is to return to a straight stage by an elastic force when the flexible plate portion 302 is folded into a state shown in Fig. 19A to Fig. 19E, and therefore, a fitted state to the outer sheath case 212 is easily kept.

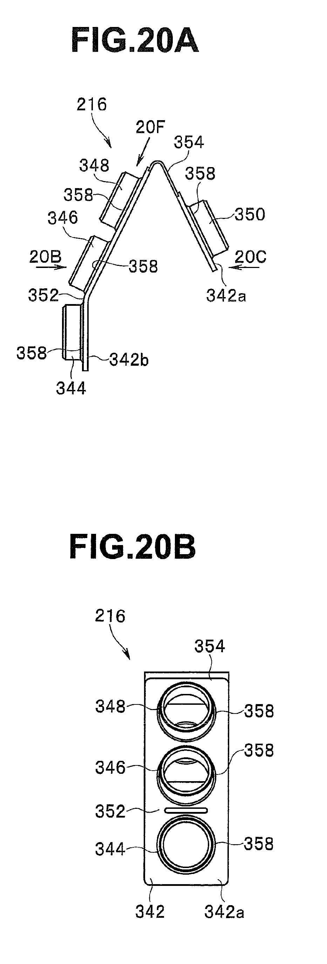

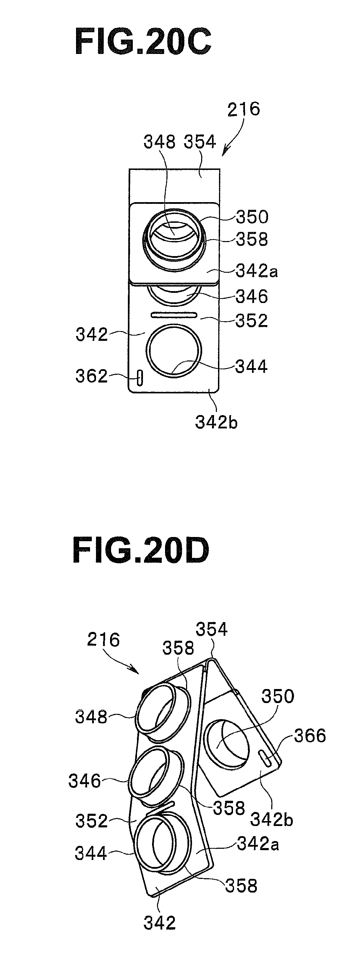

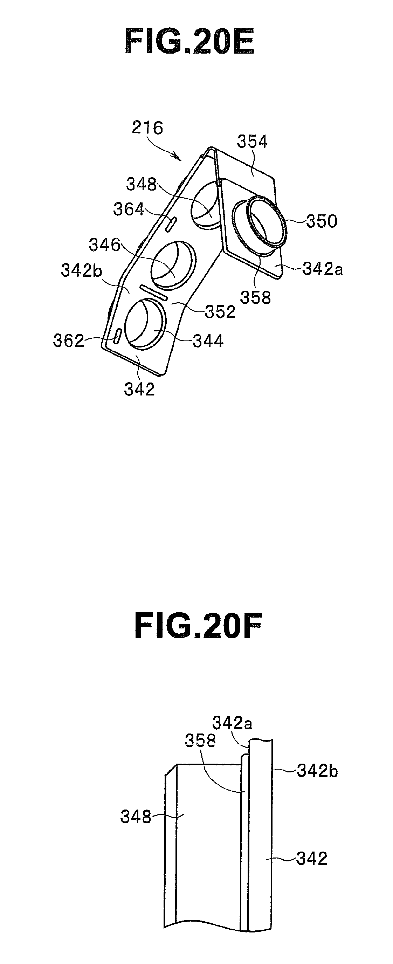

In positions (the back surface 302b side) at opposite sides from the protruding directions of the respective pressing portions 304, 306, 308 and 310, at boundaries of the flexible plate portion 302 and the button portions 312, of the proximal portions 312a of the respective button portions 312, ring-shaped concave portions (first engaging portions) 324 shown in Fig. 19G, into which ring-shaped convex portions (first engaging portions) 358 (see Fig. 20F) that will be described later of the intermediate member 216 are fitted are formed. The ring-shaped concave portion 324 is preferably formed to have a corner portion in a rectangular shape as much as possible. Note that corner portions of the ring-shaped concave portion 324 and the ring-shaped convex portion 358 are preferably formed to have the same angles so as to be in close contact with each other on two surfaces.

The intermediate member 216 shown in Fig. 20A to Fig. 20F is formed into a substantially rectangular shape from a rigid resin material having heat resistance and electrical insulating properties such as polypropylene, for example. The intermediate member 216 is formed from a material that is more rigid than that of the flexible plate 214, and is formed from the material that is more flexible than the outer sheath case 212.

As shown in Fig. 20A to Fig. 20E, the intermediate member 216 has the rigid plate portion 342, and the cylindrical collars (the first to the fourth collars) 344, 346, 348 and 350 as four ring-shaped edge portions in the embodiment. One side surface (front surface) 342a of the rigid plate portion 342 presses the flexible plate portion 302 of the flexible plate 214 between the one side surface 342a and the first case raised portion 234a, between the one side surface 342a and the first case slant surface portion 234b, and between the one side surface 342a and the second case slant surface portion 236b respectively. Namely, the rigid plate portion 342 of the intermediate member 216 presses an inside of the flexible plate portion 302 of the flexible plate 214 toward an outside. A site (a boundary) corresponding to the first case raised portion 234a and the first case slant surface portion 234b, of the rigid plate portion 342, has a first folded portion 352 that is foldable. A site (a boundary) corresponding to the first case slant surface portion 234b and the second case slant surface portion 236b has a second folded portion 354 that is foldable.

The first and the second folded portions 352 and 354 are formed by, for example, being thinned or the like.

The first collar 344 supports an inter circumferential face of the button portion 312 of the first pressing portion 304 of the flexible plate 214 between the first collar 344 and the first electric switch opening 272 of the outer sheath case 212. The second collar 346 supports an inner circumferential face of the button portion 312 of the second pressing portion 306 of the flexible plate 214 between the second collar 346 and the second electric switch opening 274 of the outer sheath case 212. The third collar 348 supports an inner circumferential face of the button portion 312 of the third pressing portion 308 of the flexible plate 214 between the third collar 348 and the third electric switch opening 276 of the outer sheath case 212, and the fourth collar 350 supports an inner circumferential face of the button portion 312 of the fourth pressing portion 310 of the flexible plate 214 between the fourth collar 350 and the fourth electric switch opening 278 of the outer sheath case 212. In particular, the first to the fourth collars 344, 346, 348 and 350 sandwich the proximal portions 312a of the button portions 312 between the first to the fourth collars 344, 346, 348 and 350, and the first to the fourth electric switch openings 272, 274, 276 and 278.

In boundaries of the rigid plate portion 342 and the respective collars 344, 346, 348 and 350, the ring-shaped convex portions 358 shown in Fig. 20F on which the ring-shaped concave portions 324 (see Fig. 19G) of the flexible plate 214 are fitted are formed. The ring-shaped convex portion 358 is preferably formed to have a rectangular corner portion as much as possible so as to be fitted in the ring-shaped concave portion 324.

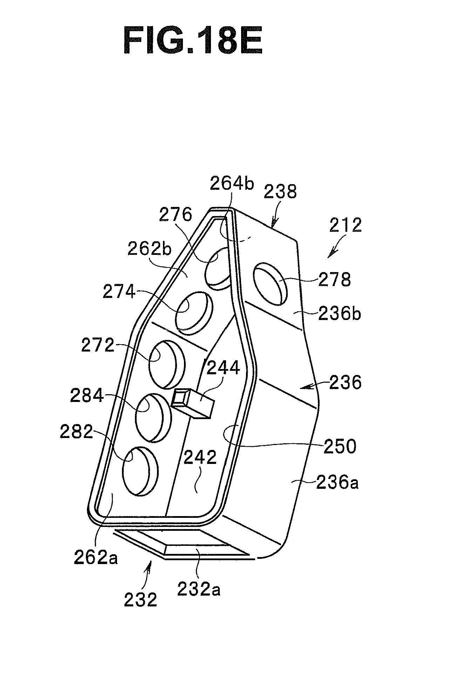

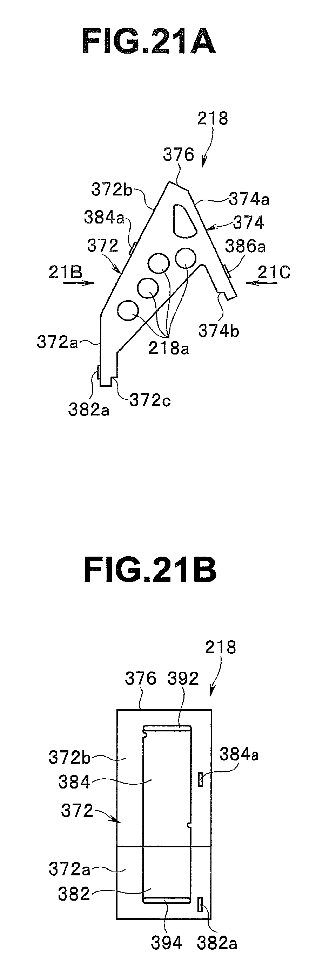

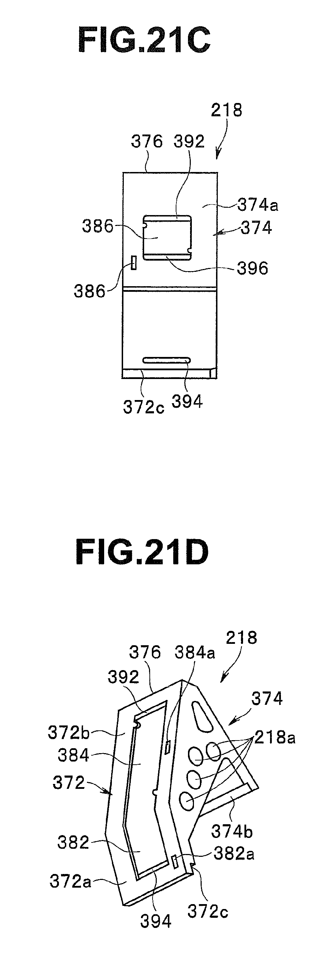

As shown in Fig. 20C to Fig. 20E, in the other side surface (the back surface) 342b of the rigid plate portion 342 of the intermediate member 216, concave-shaped portions (engaging portions) 362, 364 and 366 are formed, in which convex-shaped portions (engaging portions) 382a, 384a and 386a (see Fig. 21A to Fig. 21E) of the holder 218 are respectively fitted. The concave-shaped portions (the engaging portions) 362, 364 and 366 are preferably formed at a side in close vicinity to the opening 250 by being separated from one another on the bottom portion 242 (see Fig. 18A, Fig. 18D and Fig. 18E) of the outer sheath case 212 in the state in which the intermediate member 216 is disposed in the outer sheath case 212.

The holder 218 shown in Fig. 21A to Fig. 21E is formed from a rigid resin material having heat resistance and electrical insulating properties such as polypropylene, for example. The holder 218 is formed from a material more rigid than the flexible plate 214 and the intermediate member 216, and is formed from the material having substantially the same rigidity as that of the outer sheath case 212. Namely, the outer sheath case 212 and the holder 218 may have the same rigidity, or may be in any one of a state in which the outer sheath case 212 is more rigid than the holder 218, and a state in which the outer sheath case 212 is more flexible than the holder 218.

An outer periphery of the holder 218 is formed into a shape corresponding to a side in close vicinity to the top portion 238 of the first and the second case side portions 234 and 236 of the outer sheath case 212. The holder 218 has first and second holder side portions (holder outer circumferential faces) 372 and 374. The first and the second holder side portions 372 and 374 are integrally connected at a top portion 376.

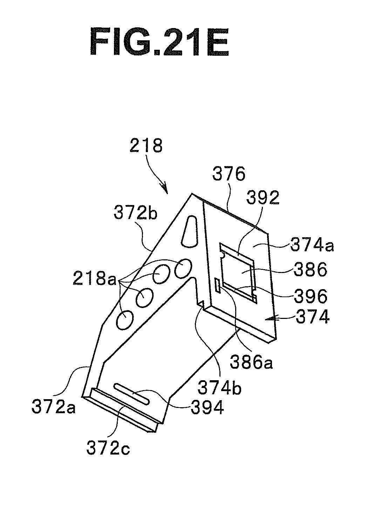

Here, the holder 218 may be formed into a substantially V-shape having the first and the second holder side portions 372 and 374 depending on selection of the material, but in order to exhibit a pressing force to the inner circumferential faces 262a, 262b and 264b of the first and the second case side portions 234 and 236 of the outer sheath case 212, the holder 218 is preferably in a block shape having an integral material in a portion between the first and the second holder side portions 372 and 374 in addition to the first and the second holder side portions 372 and 374. Note that in the embodiment, for the purpose of reduction in weight of the holder 218, a plurality of circular holes 218a (see Fig. 21A, Fig. 21D and Fig. 21E) are preferably formed in the portion between the first and the second holder side portions 372 and 374.

The first holder side portion 372 has a first holder raised portion 372a along the first case raised portion 234a, and a first holder slant surface portion 372b that is inclined toward the top portion 376 from the first holder raised portion 372a along the first case slant surface portion 234b. The second holder side portion 374 has a second holder slant surface portion 374a along the second case slant surface portion 236b.

An angle that is formed by the slant surface portions 372b and 374a of the first and the second holder side portions 372 and 374 is the same or substantially the same as an angle that is formed by the inner circumferential faces 262b and 264b of the first and the second case slant surface portions 234b and 236b of the outer sheath case 212.

In the first holder raised portion 372a, a first fitting portion 372c in which the first column 244 of the outer sheath case 212 is fitted is formed, and in the second holder slant surface portion 374a, a second fitting portion 374b in which the second column 246 of the outer sheath case 212 is fitted is formed.

Here, when the holder 218 is fitted to the columns 244 and 246 of the outer sheath case 212, slight clearances are respectively formed between the first case raised portion 234a and the first holder raised portion 372a, between the first case slant surface portion 234b and the first holder slant surface portion 372b, and between the second case slant surface portion 236b and the second holder slant surface portion 374a. Sizes of the clearances are formed to be slightly smaller than a total thickness of a thickness of the flexible plate portion 302 of the flexible plate 214 and a thickness of the rigid plate portion 342 of the intermediate member 216.

Note that the first column 244 of the outer sheath case 212 has a surface facing the first case raised portion 234a and a surface facing the first case slant surface portion 234b so as to exert a force of the first holder side portion 372 of the holder 218 onto the inner circumferential face 262a of the first case raised portion 234a of the outer sheath case 212, and the inner circumferential face 262b of the first case slant surface portion 234b respectively. Further, the second column 246 of the outer sheath case 212 has a surface facing the second case slant surface portion 236b of the outer sheath case 212 so as to exert a force of the second holder side portion 374 of the holder 218 onto the inner circumferential face 264b of the second case slant surface portion 236a. Further, the second column 246 has a surface facing the top portion 238 so as to exert a force of the second holder side portion 374 of the holder 218 onto the top portion 238 of the outer sheath case 212. Therefore, the holder 218 can press the intermediate member 216 and the flexible plate 214 to the inner circumferential faces 262a, 262b and 264b of the outer sheath case 212.

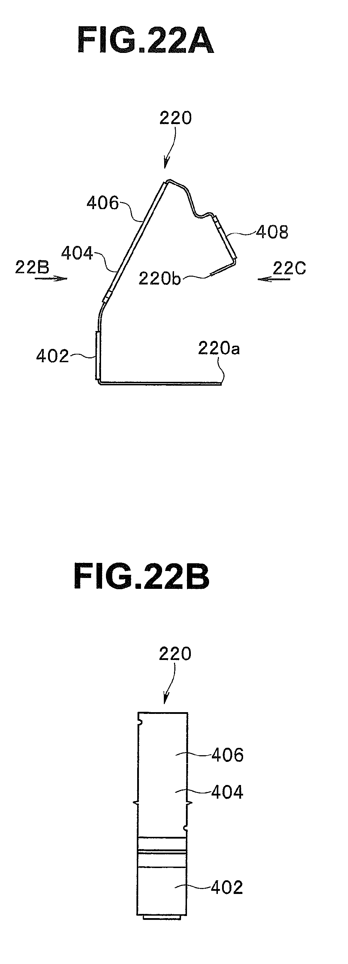

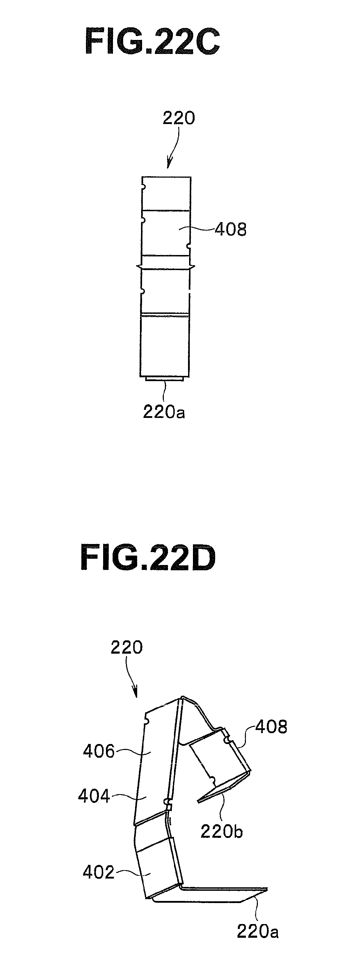

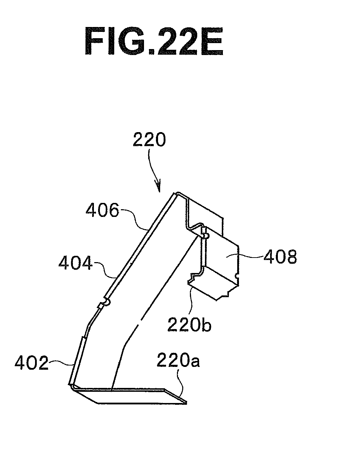

The board 220 shown in Fig. 22A and Fig. 22E is preferably formed into a flexible band shape from, for example, a polyimide resin material, a polyethylene resin material or the like. In this embodiment, the board 220 is fitted to the holder 218 so as to be wound around the holder 218. Namely, the holder 218 is used for pressing the intermediate member 216 and the flexible plate 214 to the inner circumferential faces 262a, 262b and 264b of the outer sheath case 212, and holding the board 220.

The first holder side portion 372 of the holder 218 has a first holding surface 382 that is formed into a concave shape in the first holder raised portion 372a, and a second holding surface 384 that is formed continuously to the first holding surface 382 and is formed into a concave shape in the first holder slant surface portion 372b. The second holder side portion 374 of the holder 218 has a third holding surface 386 that is formed into a concave shape in the first holder slant surface portion 372b. The second holding surface 384 and the third holding surface 386 communicate with each other in a communication passage 392 in an inside of the holder 218. At an end portion (an end portion at a side separated with respect to the top portion 376) of the first holding surface 382, a first guide passage 394 that allows one end 220a of the board 220 to advance inward of the holder 218 is formed, and at an end portion (an end portion at a side separated with respect to the top portion 376) of the third holding surface 386, a second guide passage 396 that allows the other end 220b of the board 220 to advance inward of the holder 218 is formed.

As shown in Fig. 21A to Fig. 21E, at the first holder raised portion 372a, the first holder slant surface portion 372b and the second holder slant surface portion 374a of the holder 218, the convex-shaped portions (the engaging portions) 382a, 384a and 386a on which the concave-shaped portions (the engaging portions) 362, 364 and 366 (see Fig. 20C to Fig. 20E) of the back surface 342b of the rigid plate portion 342 of the intermediate member 216 are respectively fitted are formed. The convex-shaped portions 382a, 384a and 386a are preferably formed on the bottom portion 242 (see Fig. 18A, Fig. 18D and Fig. 18E) of the outer sheath case 212 to be separated and at a side in close vicinity to the opening 250 in the state in which the holder 218 is disposed in the outer sheath case 212.

Note that in a state in which the board 220 is wound around the holder 218, a first switch 402 of the board 220 is placed on the first holding surface 382, a second and a third switches 404 and 406 of the board 220 are placed on the second holding surface 384, and a fourth switch 408 of the board 220 is placed on the third holding surface 386. At this time, due to flexibility of the board 220, the board 220 is disposed on the holder 218 so as not to protrude from the first holder side portion 372 of the holder 218 even in a boundary portion of the first holding surface 382 and the second holding surface 384.

Next, an assembling method and an operation of the switch portion 206 of the endoscope 112 of the embodiment will be described.

First, the board 220 is wound around the holder 218.

The flexible plate 214 is fitted to the outer sheath case 212. At this time, the first pressing portion 304 of the flexible plate 214 is fitted into the first electric switch opening 272 of the outer sheath case 212. Similarly, the second pressing portion 306 is fitted into the second electric switch opening 274, the third pressing portion 308 is fitted into the third electric switch opening 276, and the fourth pressing portion 310 is fitted into the fourth electric switch opening 278. Further, the ring-shaped protrusions 322 of the flexible plate 214 are caused to abut on the inner circumferential faces 262a, 262b and 264b of the outer sheath case 212. At the back surface 302b side (a side opposite to the side where the pressing portions 304, 306, 308 and 310 are protruded) of the plate portion 302 of the flexible plate 214, a space where the intermediate member 216 and the holder 218 are disposed is present.

Next, the intermediate member 216 is fitted to the flexible plate 214 in the above state. At this time, the first collar 344 of the intermediate member 216 is fitted to the first pressing portion 304 of the flexible plate 214. Similarly, the second collar 346 is fitted to the second pressing portion 306, the third collar 348 is fitted to the third pressing portion 308, and the fourth collar 350 is fitted to the fourth pressing portion 310. Further, the ring-shaped convex portions 358 of the intermediate member 216 are fitted in the ring-shaped concave portions 324 of the flexible plate 214. At this time, the collars 344, 346, 348 and 350 of the intermediate member 216 are connected to one another at the plate portion 342 and are not separated, and therefore, movement thereof in the circumferential direction is restricted. The concave-shaped portions (the engaging portions) 362, 364 and 366 of the plate portion 342 of the intermediate member 216 are located in positions in closer vicinity to the opening 250 of the outer sheath case 212 than the bottom portion 242 of the outer sheath case 212. Further, at the back surface 342b side of the plate portion 342 of the intermediate member 216 (at the opposite side from the side where the collars 344, 346, 348 and 350 are protruded), the space in which the holder 218 is disposed is present.

The holder 218 around which the board 220 is wound is disposed to press the intermediate member 216 and the flexible plate 214 to the inner circumferential faces 262a, 262b and 264b of the outer sheath case 212. At this time, the convex-shaped portions (the fitting portions) 382a, 384a and 386a of the holder 218 are fitted in and engaged in the concave-shaped portions (the fitting portions) 362, 364 and 366 of the plate portion 342 of the intermediate member 216. Subsequently, the holder 218 is pressed to the intermediate member 216 with the columns 244 and 246, and the flexible plate 214 is pressed to the inner circumferential faces 262a, 262b and 264b of the outer sheath case 212 with the intermediate member 216.

At this time, as shown in Fig. 17A, the convex portion 314 of the first pressing portion 304 is disposed in an inside of the first collar 344 to face the first switch 402, the convex portion 314 of the second pressing portion 306 is disposed in an inside of the second collar 346 to face the second switch 404, the convex portion 314 of the third pressing portion 308 is disposed in an inside of the third collar 348 to face the third switch 406, and the convex portion 314 of the fourth pressing portion 310 is disposed in an inside of the fourth collar 350 to face the fourth switch 408. Therefore, when, for example, the button portion 312 of the first pressing portion 304 is pressed, the first switch 402 is switched, and a proper operation settable in the processor 118 can be performed. For example, the first switch 402 can be used as a release switch for an observation image of the endoscope 112. Besides, the first to the fourth switches 402, 404, 406 and 408 can be properly set as switches for image recording, image printing, and observation using a light different from observation by a white color light and the like. Like this, the switches 402, 404, 406 and 408 can be set in accordance with a use state by known means that is provided in, for example, the processor 118 or the like.

Note that the opening 250 of the outer sheath case 212 is hermetically sealed with the lid member 252 shown in Fig. 18D.

Accordingly, without using a screw or an adhesive, the flexible plate 214, the intermediate member 216, and the holder 218 around which the board 220 is wound can be fixed to the predetermined positions with respect to the outer sheath case 212. At this time, the switch portion 206 can be formed by simple assembling work of disposing the flexible plate 214 having the plurality of button portions 312, the intermediate member 216 having the plurality of collars 344, 346, 348 and 350, and the holder 218 around which the board 220 having the plurality of switches 402, 404, 406 and 408 is wound in the outer sheath case 212.

As shown in Fig. 17B, the ring-shaped protrusions 322 of the front surface 302a of the flexible plate portion 302 of the flexible plate 214 are in close contact with the inner circumferential faces 262a, 262b and 264b of the outer sheath case 212, and the ring-shaped protrusions 322 of the flexible plate 214 elastically deform to perform a function like O-rings. Therefore, a liquid such as water can be prevented from penetrating into between the button portions 312 of the flexible plate 214 and the inner circumferential faces 262a, 262b and 264b of the outer sheath case 212. Accordingly, even if cleaning, high pressure steam sterilization and the like that are performed to reuse the endoscope 112 are performed, a liquid such as water can be prevented from penetrating into between the button portions 312 of the flexible plate 214 and the inner circumferential faces 262a, 262b and 264b of the outer sheath case 212. Namely, the endoscope 112 of the embodiment can endure cleaning, high pressure steam sterilization and the like that are performed to reuse the endoscope 112.

Further, the ring-shaped concave portion 324 of the flexible plate 214 and the ring-shaped convex portion 358 of the intermediate member 216 are fitted in the shapes having the rectangular corner portions as much as possible. Therefore, the ring-shaped concave portion 324 and the ring-shaped convex portion 358 are in close contact with each other on both of the surfaces 324a and 358a that are parallel with the flexible plate portion 302 of the flexible plate 214 and the rigid plate portion 342 of the intermediate member 216, and the surfaces 324b and 358b that are perpendicular to the flexible plate portion 302 and the rigid plate portion 342. Accordingly, the ring-shaped concave portion 324 and the ring-shaped convex portion 358 can be brought into close contact with two ring-shaped surfaces. Even if one of the button portions 312 is deformed by being pressed and pulled, the other button portions 312 can keep in the same shapes in the same positions.

Note that as shown in Fig. 17B, a distance β between a corner portion that is formed by the electric switch opening 278 of the outer sheath case 212 and the inner circumferential face (the second inner circumferential face) 264b of the second case slant surface portion 236b, and a corner portion that is formed by the surface 324a that is parallel with the flexible plate portion 302 of the flexible plate 214 and the surface 324b that is perpendicular to the flexible plate portion 302 is formed to be smaller than a thickness α of the flexible plate portion 302 of the flexible plate 214. The same applies to relations of the other electric switch openings 272, 274 and 276 of the outer sheath case 212, the other pressing portions 304, 306 and 308 of the flexible plate 214, and the other collars 344, 346 and 348 of the intermediate member 216.

The convex-shaped portions (the fitting portions) 382a, 384a and 386a of the holder 218 are fitted in and engaged in the concave-shaped portions (the fitting portions) 362, 364 and 366 of the plate portion 342 of the intermediate member 216. Therefore, positional displacement in which the plate portion 342 of the intermediate member 216 moves to the opening 250 side and the bottom surface 242 side of the outer sheath case 212 with respect to the holder 218 can be prevented. Further, positional displacement in which the plate portion 342 of the intermediate member 216 moves in a longitudinal direction with respect to the holder 218 also can be prevented. Note that the collars 344, 346, 348 and 350 of the intermediate member 216 are connected to one another at the plate portion 342, and are not separated from one another, and therefore, movement in the circumferential direction around the center axis C (see Fig. 17A) is restricted. However, with respect to the holder 218, the positional displacement in which the plate portion 342 of the intermediate member 216 moves with respect to the outer sheath case 212 can be prevented, whereby the collars 344, 346, 348 and 350 can be prevented from rotating in the circumferential direction around the center axis C respectively.

When, for example, the button portion 312 of the fourth pressing portion 310 is pressed along the center axis C thereof, a force is applied so that the flexible plate portion 302 that is integrally formed in the button portion 312 expands with respect to the center axis C. At this time, the fourth collar 350 of the intermediate member 216 is rigid to the flexible plate 214, and the fourth collar 350 hardly deforms. Therefore, of the ring-shaped concave portion 324 of the flexible plate 214, the surface 324a parallel with the flexible plate portion 302 presses the surface 358a parallel with the rigid plate 342 of the ring-shaped convex portion 358 of the intermediate member 216. However, with respect to the flexible plate 214, the intermediate member 216 is formed from a rigid resin material, the button portion 312 is sandwiched between the fourth electric switch opening 278 of the outer sheath case 212, and the fourth collar 350 of the intermediate member 216, and between the inner circumferential faces 262a, 262b and 264b of the outer sheath case 212, and the flexible plate 214, the ring-shaped protrusions 322 of the flexible plate 214 are in close contact with the inner circumferential faces 262a, 262b and 264b. Therefore, deformation of the button portion 312 is performed in a position at a side protruded with respect to the outer surface of the outer sheath case 212 along the center axis C from the fourth collar 350, and movement in a direction to expand with respect to the center axis C is prevented. Namely, deformation of the button portion 312 is performed mainly by the deformation portion 312b deforming instead of the proximal portion 312a.

When the button portion 312 is pulled to the protruded side along the center axis C thereof, the button portion 312 is pulled, whereby the flexible plate portion 302 is pulled to the center axis C side to shrink. At this time, the surface 324b that is perpendicular to the flexible plate portion 302 of the ring-shaped concave portion 324 of the flexible plate 214 presses the surface 358b perpendicular to the rigid plate 342 of the ring-shaped convex portion 358 of the intermediate member 216. However, in contrast with the flexible plate 214, the intermediate member 216 is formed from a rigid resin material, and therefore, the surface 324b perpendicular to the flexible plate portion 302 of the ring-shaped concave portion 324 of the flexible plate 214 is prevented from moving toward the center axis C side.

Further, when the button portion 312 is pressed from a direction out of the center axis C, the button portion 312 is pressed, whereby the pressed side expands, and the opposite side thereof shrinks. As for the shrunk side of the button portion 312, shrinkage occurs in a position at the side that is protruded with respect to the outer surface of the outer sheath case 212 along the center axis C from the fourth collar 350 of the button portion 312, similarly to the time when the button portion 312 of the fourth pressing portion 310 is pressed along the center axis C thereof, for example, and movement in the direction to expand with respect to the center axis C is prevented. As for the expanded side of the button portion 312, the surface 324b perpendicular to the flexible plate portion 302 of the ring-shaped concave portion 324 of the flexible plate 214 is prevented from moving toward the center axis C side, and movement is also restricted since the ring-shaped concave portion 288 and the ring-shaped protrusion 322 are fitted to each other between the outer sheath case 212 and the flexible plate 214, similarly to the time when the button portion 312 of the fourth pressing portion 310 is pressed along the center axis C (see Fig. 17A) thereof, for example. Accordingly, when the button portion 312 is pulled in the direction to be out of the center axis C, the button portion 312 keeps the state shown in Fig. 17A, and is not easily detached from the fourth electric switch opening 278.

The above contents similarly apply to the first to the third pressing portions 304, 306 and 308.

As described above, in the operation portion 132 of the endoscope 112 according to the embodiment, the flexible plate 214 that is flexible, the rigid intermediate member 216, and the rigid holder 218 are sequentially disposed with respect to the rigid outer sheath case 212, whereby the switch portion 260 can be formed. When the switch portion 206 is formed in this manner, water tightness between the outer sheath case 212 and the flexible plate 214 is achieved, and a liquid can be prevented from penetrating into the switch portion 206. Further, with respect to the rigid outer sheath case 212, the flexible plate 214 that is flexible, and the rigid intermediate member 216 and the rigid holder 218 are sequentially disposed, whereby when various forces are applied to the button portions 312, positional displacement of the button portions 312 can be prevented.

Note that in the embodiment, the example in which the first pressing portion 304 is disposed in the first raised portion 234a of the outer sheath case 212 as shown in Fig. 17A is described, but the pressing portion does not have to be disposed in the first raised portion 234a of the outer sheath case 212. Further, the example in which the second and the third pressing portions 306 and 308 are disposed in the first slant surface portion 234b of the outer sheath case 212 is described, but the pressing portions do not have to be disposed in the first slant surface portion 234b of the outer sheath case 212. Further, the example in which the fourth pressing portion 310 is disposed in the second slant surface portion 236b of the outer sheath case 212 is described, but the pressing portion does not have to be disposed in the second slant surface portion 236b of the outer sheath case 212. Namely, various states can be allowed for disposition of the pressing portions to the outer sheath case 212.

Further, the case in which the electric switch portions 156 are formed at the outer sheath case 212 is described in the embodiment, but the air/water feeding button 152 and the suction button 154 can be formed in the state shown in Fig. 16 by a known method.

Next, various modifications of the second embodiment will be described with use of the drawings. In the modifications that will be described as follows, the same members or the members having the same functions as the members described in the second embodiment will be assigned with the same reference signs as much as possible, and the detailed explanation will be omitted.

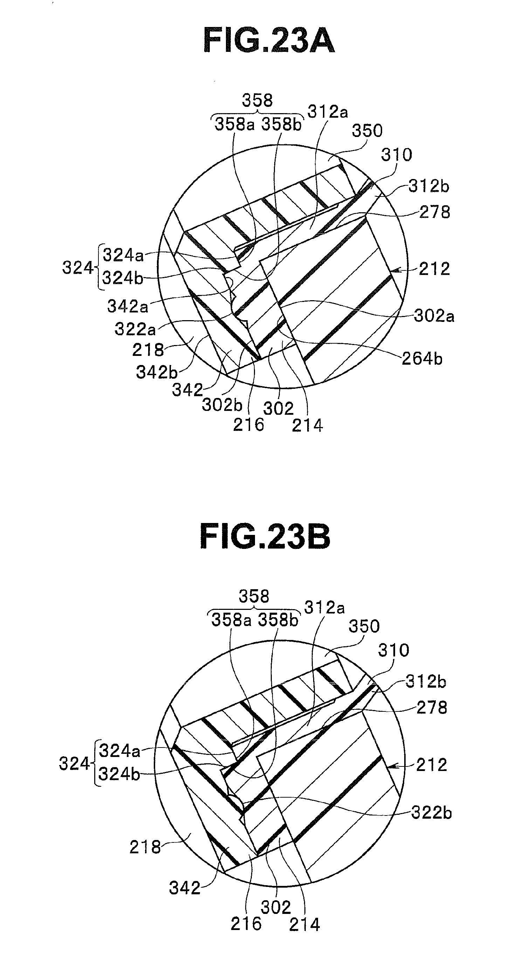

Fig. 23A shows an example in which the ring-shaped protrusions 322 of the front surface 302a of the flexible plate portion 302 of the flexible plate 214 are removed, and ring-shaped protrusions 322a are formed on the back surface 302b of the flexible plate portion 302. In this case, the ring-shaped protrusion 322a of the back surface 302b of the flexible plate portion 302 of the flexible plate 214 presses the front surface 342a of the rigid plate portion 342 of the intermediate member 216. At this time, since the rigid plate portion 342 of the intermediate member 216 is more rigid than the flexible plate portion 302 of the flexible plate 214, the deformation amount of the rigid plate portion 342 is small, and the rigid plate portion exhibits a force to cause the flexible plate portion 302 to press the inner circumferential face 264b of the outer sheath frame 212.

Fig. 23B shows an example in which the ring-shaped protrusions 322 are removed from the flexible plate 214, and ring-shaped protrusions 322b are formed on the front surface 342a of the rigid plate portion 342 of the intermediate member 216. In this case, the back surface 302b of the flexible plate portion 302 of the flexible plate 214 is pressed by the ring-shaped protrusions 322b of the front surface 342a of the rigid plate portion 342. At this time, since the rigid plate portion 342 of the intermediate member 216 is more rigid than the flexible plate portion 302 of the flexible plate 214, the deformation amount of the rigid plate portion 342 is small, and the flexible plate portion 302 deforms more significantly than the rigid plate portion 342, whereby the rigid plate portion 342 exhibits a force to cause the flexible plate portion 302 to press the inner circumferential face 264b of the outer sheath frame 212.

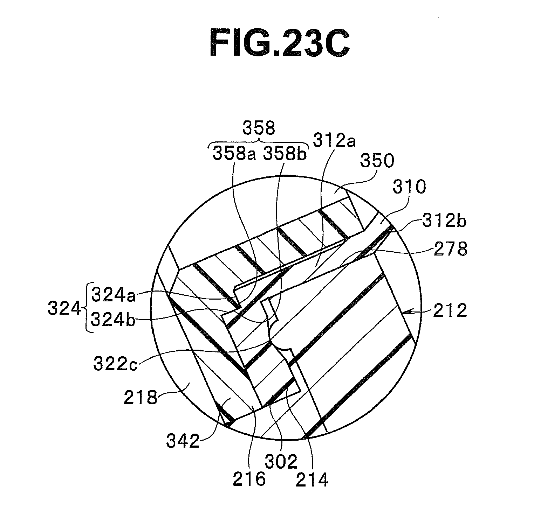

Fig. 23C shows an example in which the ring-shaped protrusions 322 are removed from the flexible plate 214, and ring-shaped protrusions 322c are formed at the inner circumferential face 264b of the outer sheath case 212. In this case, the back surface 302b of the flexible plate portion 302 of the flexible plate 214 is pressed by the ring-shaped protrusions 322c of the inner circumferential face 264b of the outer sheath case 212. At this time, since the outer sheath case 212 is more rigid than the flexible plate portion 302 of the flexible plate 214, the deformation amount of the outer sheath case 212 is small, and the flexible plate portion 302 deforms more significantly than the outer sheath case 212. Therefore, the ring-shaped protrusions 322c of the outer sheath case 212 are in close contact with the front surface 302a of the flexible plate portion 302.

Even if the ring-shaped protrusions are formed as shown in Fig. 23A to Fig. 23C, the similar effect to the case of use of the ring-shaped protrusions 322 from the flexible plat 214 described in the first embodiment can be obtained.

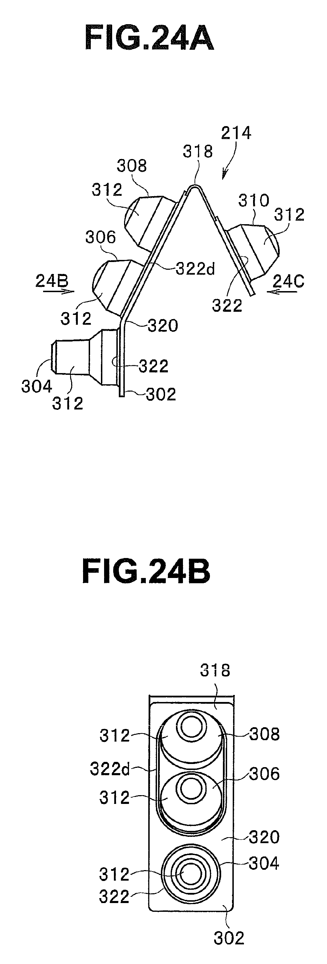

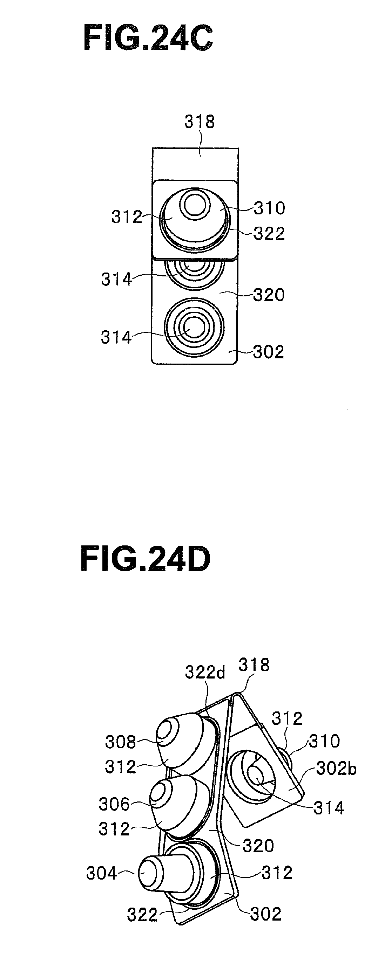

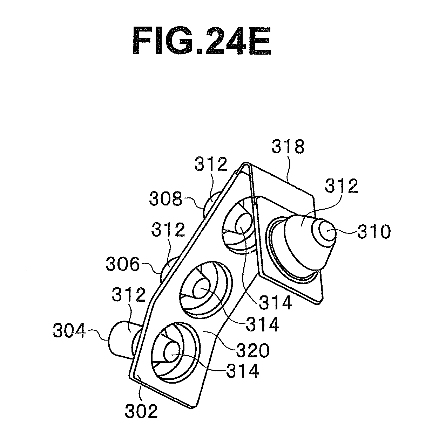

Fig. 24A to Fig. 24E show an example in which a ring-shaped protrusion 322d that is different from the ring-shaped protrusions 322 that are formed to surround the pressing portions 306 and 308 of the flexible plate 214 shown in Fig. 19A to Fig. 19F respectively is formed to surround the outer sides of the two pressing portions 306 and 308 together. The ring-shaped protrusion 322d is formed in this manner, whereby the two adjacent pressing portions 306 and 308 shown in Fig. 24A to Fig. 24D can be made closer to each other than the case shown in Fig. 19A to Fig. 19E.

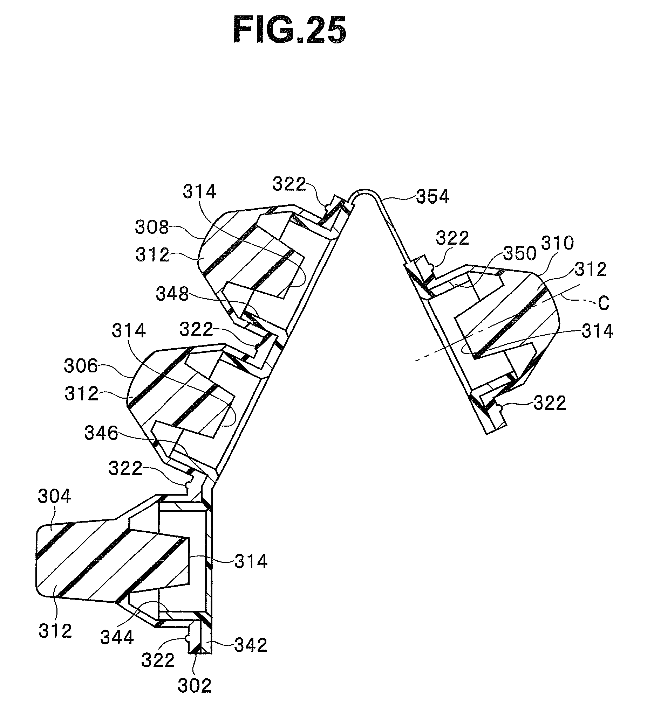

Fig. 25 shows an example in which the flexible plate 214 and the intermediate member 216 are integrally molded. When the flexible plate 214 and the intermediate member 216 are integrally molded, insert molding or the like, for example, is used. By adoption of the structure like this, the flexible plate 214 and the intermediate member 216 can be fitted to the outer sheath case 212 at a time, and therefore, assembly can be facilitated. Further, in this case, the state in which the back surface 302b of the flexible plate portion 302 of the flexible plate 214 and the front surface 342a of the rigid plate portion 342 of the intermediate member 216 are in close contact with each other is kept. Therefore, the ring-shaped concave portion 324 of the flexible plate 214 and the ring-shaped convex portion 358 of the intermediate member 216 do not have to be formed. Accordingly, it can be made unnecessary to consider positional displacement between the flexible plate 214 and the intermediate member 216.

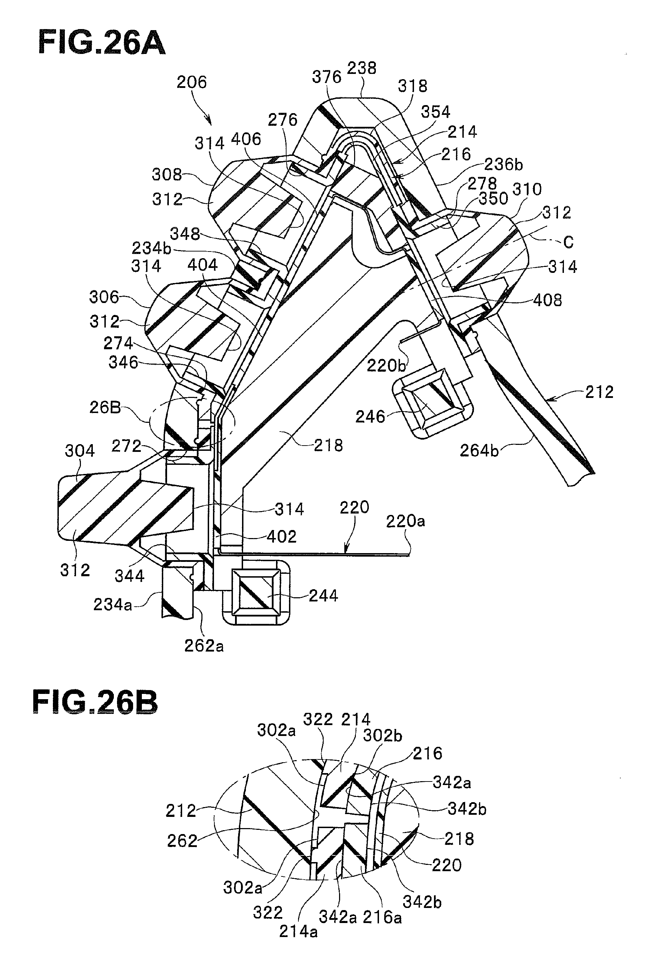

Fig. 26A shows an example in which the pressing portion 304 is made additionally fittable to the outer sheath case 212.

As shown in Fig. 26A, in a modification, the flexible plate 214 has the three pressing portions (the first to the third pressing portions) 306, 308 and 310. The intermediate member 216 has the cylindrical collars (the first to the third collars) 346, 348 and 350 as the three ring-shaped edge portions.

Here, in the modification, another flexible plate 214a that is adjacent to the flexible plate 214 is included. The flexible plate 214a has, for example, one (or a plurality of) pressing portion 304. Another intermediate member 216a that is adjacent to the intermediate member 216 is included. The intermediate member 216a has one (or a plurality of) collar 344.

If the electric switch opening 272 is formed in the outer sheath frame 212, the flexible plates 214 and 214a, the intermediate members 216 and 216a, and the holder 218 around which the board 220 of the electric switch 156 is wound are placed into the space that is formed by the inner circumferential faces 262a, 262b and 264b of the outer sheath frame 212, as shown in Fig. 26A, whereby the switch can be assembled as described in the first embodiment.

At this time, clearances may be formed between the flexible plates 214 and 214a, and between the intermediate members 216 and 216a as shown in Fig. 26B, or end portions may be in close contact with each other.

For example, the one pressing portion 304 of the flexible plate 214a, and the three pressing portions 306, 308 and 310 of the flexible plate 214 can be easily formed with use of other shapes and other raw materials. Therefore, rigidity of the pressing portions 304, 306, 308 and 310 can be adjusted depending on the position of the operation portion 132. Namely, the operation sensing of the pressing portions 304, 306, 308 and 310 can be adjusted. Further, by adjustment of the thicknesses of the pressing portion 304 and the pressing portions 306, 308 and 310, the operation sensing also can be adjusted. Furthermore, the orientation of the center axis C of the pressing portion 304 is changed to various directions without being kept in only the state shown in Fig. 26A, and thereby the operation sensing of the pressing portion 304 can be adjusted.

Accordingly, after the plurality of pressing portions 306, 308, 310 and the like are fitted to the operation portion 132, the pressing portion 304 can be added. Further, a pressing portion having different properties from the adjacent plurality of pressing portions 306, 308 and 310 can be easily added in such a manner that the thickness and the rigidity of the pressing portion 304 are changed with respect to the other plurality of pressing portions 306, 308 and 310. In this case, the pressing portion is additionally formed, and therefore, molding of the pressing portion can be easily performed.

One embodiment including the plurality of modifications is specifically described with reference to the drawings thus far, but the invention is not limited to the aforementioned embodiments, and includes all embodiments that are performed within the range without departing from the gist of the invention.

### (Appendixes)

According to the above described embodiment of the present invention as described in detail above, the configuration as follows can be obtained.

### (Appendix 1)

An endoscope, comprising:
an operation portion that is operated in a state grasped by a hand of a user; and
an insertion portion that is extended from the operation portion, and is inserted into a pore,
wherein the operation portion has
an outer sheath case that has a first and a second inner circumferential faces that face each other, and a plurality of openings that are placed in at least one of the first and the second inner circumferential faces, and is grasped with the hand of the user,
a flexible plate that has a plurality of pressing portions that are protruded through the openings to an outside from an inside of the outer sheath case and are elastically deformable, and is disposed along the first and the second inner circumferential faces of the outer sheath case,
a plate-shaped intermediate member that is disposed in the inside of the flexible plate along the first and the second inner circumferential faces of the outer sheath case, and supports the pressing portions of the flexible plate respectively to prevent a liquid from penetrating into between the outer sheath case and the flexible plate, and
a holder that holds a board having a switch that is switchable to a position where the switch is pressed by the pressing portion and a position where the switch is released with respect to the pressing portion, in a position facing the pressing portion, presses the intermediate member and the flexible plate by the intermediate member toward the first and the second inner circumferential faces of the outer sheath case to bring the intermediate member into close contact with the flexible plate and bring the flexible plate into close contact with the first and the second inner circumferential faces, and is supported in the inside of the outer sheath case.

### (Appendix 2)

The endoscope according to appendix 1,
wherein the intermediate member has a ring-shaped edge portion that holds a state in which the pressing portion of the flexible plate is caused to face the board that is held by the holder, and has
ring-shaped engaging portions that are provided at the ring-shaped edge portion, and a proximal portion of the pressing portion of the flexible plate that faces the ring-shaped edge portion to be engaged with each other to restrict deformation of the proximal portion of the pressing portion.

### (Appendix 3)

The endoscope according to appendix 1,
wherein at least one of the inner circumferential face of the outer sheath case, a front surface or a back surface of the flexible plate, and a front surface of the intermediate member has a ring-shaped convex portion that brings the front surface of the flexible plate and the inner circumferential face of the outer sheath case into close contact with each other to achieve water tightness between the front surface of the flexible plate and the inner circumferential face of the outer sheath case.

### (Appendix 4)

The endoscope according to appendix 1,
wherein the intermediate member is formed from a raw material more rigid than the flexible plate, and is formed from the raw material more flexible than the outer sheath case and the holder.

### (Appendix 5)

The endoscope according to appendix 1,
wherein the flexible plate and the intermediate member are integral with each other.

### (Appendix 6)

The endoscope according to appendix 1,
wherein the intermediate member and the holder have engaging portions that are engageable with each other.

The present application is filed claiming the priority of Japanese Patent Applications No. 2012-187849 filed in Japan on August 28, 2012 and No. 2012-090227 filed in Japan on April 11, 2012, and the above described disclosure is incorporated by reference in the present description, claims and drawings.

## Claims

1. A switch cover of a pressing type switch apparatus having a switch portion that is contained in an operation portion of a handheld device to be operated by an operator and performs a switching action by a stress having a component in a perpendicular direction with respect to an outer surface of the operation portion, the switch cover comprising:
a cylindrical portion that is placed to surround a periphery of the switch portion and extends in a direction substantially perpendicular to the outer surface of the operation portion over an inside and an outside of the operation portion;
a flexible first deformation portion that is connectively provided at a partial region in a circumferential direction of the cylindrical portion on the outside of the operation portion, and deformed by an external stress that is given by pressing;
a flexible second deformation portion that is connectively provided at a region other than the first deformation portion in the circumferential direction of the cylindrical portion on the outside of the operation portion, and deformed more easily than the first deformation portion by the external stress; and
a stress transmitting portion that is connectively provided at the first deformation portion and the second deformation portion, and transmits the stress having the component in the perpendicular direction with respect to the outer surface of the operation portion to the switch portion when the first deformation portion is deformed inward of the cylindrical portion by the external stress.

2. The switch cover according to claim 1,
wherein the stress transmitting portion integrally comprises an operation input portion in a protrusion shape in which a part of a circumferential face continuing to the first deformation portion and a top face are set as pressed surfaces, and a pressing portion that transmits the stress applied to the pressed surfaces of the operation input portion to the switch portion.

3. The switch cover according to claim 2,
wherein the second deformation portion includes an inclined portion that inclines at a larger angle than the first deformation portion, with respect to the perpendicular direction to the outer surface of the operation portion.

4. The switch cover according to claim 3,
wherein the second deformation portion forms a stepped shape between the second deformation portion and the cylindrical portion by the inclined portion.

5. The switch cover according to claim 2, wherein the operation input portion includes a stress concentration portion that causes the external stress to concentrate on a part of the second deformation portion.

6. The switch cover according to claim 5,
wherein the stress concentration portion is a corner-shaped portion that is formed on the circumferential face of the operation input portion.

7. The switch cover according to claim 2,
wherein the top face of the operation input portion inclines by having an angle of elevation from the first deformation portion side to the second deformation portion side.

8. The switch cover according to claim 2,
wherein the operation input portion has a sectional area set to be smaller than a sectional area of the cylindrical portion, and is provided to be offset to the first deformation portion side with respect to a center of the cylindrical portion.

9. The switch cover according to claim 1,
wherein a wall thickness of the second deformation portion is formed to be thinner than a wall thickness of the first deformation portion.

10. A pressing type switch apparatus comprising a switch portion that is contained in an operation portion of a handheld device to be operated by an operator and performs a switching action by a stress having a component in a perpendicular direction with respect to an outer surface of the operation portion, and a switch cover that transmits the stress to the switch portion,
wherein the switch cover includes
a cylindrical portion that is placed to surround a periphery of the switch portion, and extends in a direction substantially perpendicular to the outer surface of the operation portion over an inside and an outside of the operation portion,
a flexible first deformation portion that is connectively provided at a partial region in a circumferential direction of the cylindrical portion on the outside of the operation portion, and deformed by an external stress that is given by pressing,
a flexible second deformation portion that is connectively provided at a region other than the first deformation portion in the circumferential direction of the cylindrical portion on the outside of the operation portion, and deformed more easily than the first deformation portion by the external stress, and
a stress transmitting portion that is connectively provided at the first deformation portion and the second deformation portion, and transmits the stress having the component in the perpendicular direction with respect to the outer surface of the operation portion to the switch portion when the first deformation portion is deformed inward of the cylindrical portion by the external stress.

11. An endoscope provided with a pressing type switch apparatus including a switch portion that is contained in an operation portion to be operated by an operator and performs a switching action by a stress having a component in a perpendicular direction with respect to an outer surface of the operation portion, and a switch cover that transmits the stress to the switch portion,
wherein the switch cover comprises
a cylindrical portion that is placed to surround a periphery of the switch portion, and extends in a direction substantially perpendicular to the outer surface of the operation portion over an inside and an outside of the operation portion,
a flexible first deformation portion that is connectively provided at a partial region in a circumferential direction of the cylindrical portion on the outside of the operation portion, and deformed by an external stress that is given by pressing,
a flexible second deformation portion that is connectively provided at a region other than the first deformation portion in the circumferential direction of the cylindrical portion on the outside of the operation portion, and deformed more easily than the first deformation portion by the external stress, and
a stress transmitting portion that is connectively provided at the first deformation portion and the second deformation portion, and transmits the stress having the component in the perpendicular direction with respect to the outer surface of the operation portion to the switch portion when the first deformation portion is deformed inward of the cylindrical portion by the external stress.
